# EUROPEAN PATENT APPLICATION

(11) **EP 1 469 401 A2**
(43) Date of publication of application: **20.10.2004**
(21) Application number: 04008910.4
(22) Date of filing: 14.04.2004
(51) Int. Cl.: G06F 17/30

(54) **System and method for searching information**

(30) Priority: 14.04.2003 JP 2003109054
(71) Applicant: NEC CORPORATION, Tokyo (JP)
(72) Inventor: Nakazato, Takeru, Minato-ku, Tokyo (JP); Miyakawa, Tomoya, Minato-ku, Tokyo (JP); Kenmochi, Akihisa, Minato-ku, Tokyo (JP); Asogawa, Minoru, Minato-ku, Tokyo (JP)
(74) Representative: Betten & Resch

(57) **Abstract**

An input receiving unit (11) receives a query to a database from a user, creates a user query for searching information in accordance with the query of the user. A query generating unitC (12) generates a database query for actually searching in accordance with the user query or a re-search query for re-searching information. A searching unit (13) executes a search of information in accordance with the database query. A condition setting unit (16) has an analyzing condition and an output condition. An analyzing unit (14) analyzes an information searching result in accordance with the analyzing condition supplied from the condition setting unit (14). An output control unit (15) outputs an information analyzing result as output information in accordance with the output condition supplied from the condition setting unit (14), creates the re-search query by using the information analyzing result, and supplies the re-search query to the query generating unit (12).

## Description

The present invention relates to a system and method for searching information.

In the fields of the medicine, pharmacy, molecular biology and the like, the structures and functions of many genes and proteins have been unveiled so far because of the elucidation of the cause of diseases and that of the mechanism of the onset of diseases, the development of remedy, the biological discovery, and the like. Moreover, in order to clarify the mechanism of the biological development and growth and the biofunction at the cellular level, the study to elucidate the base sequences of genomes, namely, all DNA (deoxyribonucleic acid) has been actively conducted in connection with various species such as human, mouse, rat, nematode, rice, etc. The elucidation of the genome has progressed and the study on the protein generated according to information expressed by a gene has been rapidly developed.

By the study on the genes and the proteins, enormous data can be obtained in connection with the gene base sequence of the gene, the gene position on a chromosome, the analogous gene, the mutation, the amino acid sequence of protein, the three-dimensional structure of protein. This constructs various biological information databases including data obtained by the study.

One of the biological information databases, which are well known to the researchers and frequently used, is Entrez (http://www.ncbi.nlm.nih.gov/entrez/) of NCBI (National Center for Biotechnology Information) in America. National Institute of Genetics in Japan, EBI (European Bioinformatics Institute) of EMBL (European Molecular Biology laboratory) and the like provide the databases including mainly the sequence information of the genes and the proteins. LocusLink (http://www.ncbi.nlm.nih.gov/LocusLink) provided by NCBI, SWISS-PROT (http://kr.expasy.org/sprot/) including mainly information on the proteins are also frequently used.

Under such circumstances, it is essential for the researchers in the fields of the medicine, pharmacy, molecular biology and the like to use the aforementioned biological information databases. For example, in the study on the gene relating to the disease, there is a case in which comparison in a gene expression between a patient and an ordinary person is performed in order to specify the gene that can be used in diagnosis. At this time, it is not uncommon that an unexpected gene expression is found. In this case, it is necessary to consult detailed information on the found gene expression by use of the databases.

Moreover, in the study of searching a new gene, there is a case in which only a fragment of DNA is obtained. In this case, it is checked whether there is one that conforms to the sequence of the obtained fragment in the known DNA sequences by use of the databases, making it possible to determine whether the fragment is an unknown gene.

Furthermore, in the study on what function a protein, which is generated from a certain gene, has, the sequence of a target gene is consulted by the databases. Then, a part of the sequence is chemically synthesized to form DNA including the sequence of the target gene by a polymerase chain reaction. Moreover, a protein is generated based on the formed DNA. Then, an experiment for checking an interaction between the target gene and the function of the generated protein and an experiment for checking an electrical characteristic of the generated protein are performed.

The above shows merely a part of the examples of using the biological information databases. The researchers conduct the study while obtaining various kinds of information from the biological information databases. It is a general rule that newly obtained knowledge is registered in the database. For this reason, the biological information databases are continued to be updated everyday or every week.

A keyword (word or phase) that is used in searching by use of the biological information database is called query or query by example (QBE). The query is hereinafter used in this specification.

In the study carried out by the researchers, a great deal of weight is placed on obtaining not only information on the gene and protein but also information relating to scholarly papers, books, documents of patent publications. Particularly, reading the theses described in the academic journals issued in the world is essential to knowing the existing knowledge and the latest trend or experimental methods in the field of his/her own study.

PubMed (http://www.ncbi.nlm.nib.gov/entrez/guery.fcgi?db=PubMed) is a tool that searches a biomedical document from the biomedical document database provided to the whole world at no charge by NCBI. PubMed is extremely widely and frequently used by the researchers in the world. MEDLINE (Medical Literature, Analysis, and Retrieval System Online) as a core for the PubMed database contains more than 4600 documents published in over 70 countries including the USA in the fields of the medicine, pharmacy, molecular biology and the like. The number of languages used in these documents is over 40. Contained data from the middle of the 1960s until today is more than 11 millions. Moreover, new documents are added everyday in principle. At the time of conducting a search using PubMed, the researcher uses a gene or protein name and an author as keywords. In this case, the list of documents relating to the keywords can be obtained via PubMed. The researcher can read the summary of a journal article by following a link of an interesting document. Moreover, when the organization to which the researcher belongs has a predetermined contract with the publisher of the document, the researcher can read the whole of the journal article by further following the link.

A part of the above contents is disclosed in, for example, Current Issues in Molecular Biology, 3 (2001) pp.47 to 55 and Canadian Medical Association Journal, 164 (2001) pp. 1317 to 1319.

However, the conventional search system has the following problems. A first problem is that when only the gene or protein name, which the researcher knows, is used as the keyword, there is an omission in the searching result in some cases.

Generally, at the time of searching by use of the biological information database, the gene or protein name is used as the keyword. However, newly discovered gene and protein are uniquely named by the researchers, who discovered them, respectively. For this reason, the names are not unified as a whole. When the genes or proteins having a high homology are different in the species from each other, there is a case in which completely different names are given to them, respectively. Moreover, when a new function is discovered in the known gene or protein, there is a case in which a name appropriate to the new function is given thereto and such a name that generalizes the gene or protein having an analogous function is given thereto. In terms of these points, the gene or protein having multiple names exists and a registered name is different for each biological information database in some cases. Accordingly, even when the researcher conducts a search using the gene or protein name that he/she individually knows, the researcher cannot obtain all necessary information. Namely, there is the omission in the searching result.

Ununiformity in the format of the gene and protein names among the multiple existing biological information databases becomes a large factor that causes the omission in the searching result. For example, "Carbonic Anhydrase 1", which is one of enzymes, is written in several forms such as "CA1", "CA 1", "CA-1", etc as its abbreviation. Moreover, a Roman numeral "I" is used for an Arabic numeral "1." Accordingly, when the designated keyword does not comply with the format used in the biological information database, an omission sometimes occurs in the searching result after all.

The aforementioned omission in search causes a more serious problem in some cases. For example, when the researcher, who studies a certain gene or protein, cannot obtain data relating to a name that he/she knows from the biological database, the researcher misunderstands that the gene or protein to be studied is not registered therein. Namely, the researcher misunderstands that the gene or protein to be studied is a new gene or protein. In this case, there is a possibility that the researcher will prepare a sampling of the gene or protein to waste time, labor, and cost. In order to avoid such a problem, the researcher conducts the search using the multiple biological information databases. Then, the researcher is forced to conduct a search using another database based on another gene name as a keyword obtained from a certain database. This puts an enormous load on the researcher.

A second problem is that the gene or protein name must be used in principle at the time of conducting a search by use of several biological information databases.

When each of the gene and the protein is registered in the biological information database such as NCBI, DDBJ (DNA Data Bank of Japan) and the like, a number, which is called accession number, is given as an ID in the database. The accession number is decided according to a unique rule for each biological information database. For this reason, even in the same gene or protein, the accession number is different for each biological information database in some cases. Accordingly, at the time of conducting the search by use of the multiple biological information databases, there is a case in which difficulty occurs in conducting the search by use of the accession number.

A trial that unifies the formats of the names and the accession numbers has been started among the existing biological databases that are frequently used. However, this trial is at the present state very far from the stage of practical application. Furthermore, since many trivial names are used in the research papers, we must say that there are difficult situations.

Moreover, in many biological information databases, there is a problem in which the document cannot be searched by use of biological information (such as sequence, molecular weight, etc.) except the name. For example, in order to search the document using the accession number, the name and accession number of the searching gene or protein must be consulted in advance using the Entrez database of NCBI. This puts an enormous load on the user. Moreover, there is a case in which a name and an accession number are not given to a new gene or protein. For this reason, when a candidate for a new gene or protein is obtained, it is general that a search is first conducted by use of a base or amino acid sequence.

Furthermore, in conventional, BLAST (Basic Local Alignment Search Tool) is used as a tool for consulting the homology of the base or amino acid sequence. The researcher uses BLAST to obtain the name of gene or protein having homology. Then, the researcher manually input the obtained name as the keyword to the terminal in order to search the document by use of PubMed and the like. A series of these operations put a considerable load on the researcher.

In addition, it is assumed that the contents of the aforementioned documents are incorporated into this specification.

Accordingly, an object of the present invention is to provide an information search system and information search method that is less prone to cause an omission in a searching result even if the number of keywords is one.

Also, another object of the present invention is to provide an information search system and information search method that can execute a search regardless of the kinds of keywords.

Moreover, another object of the present invention is to provide an information search system and information search method with a small load placed on a user to conduct a search.

In order to attain the above object, an information search system according to a first aspect of the present invention includes an input receiving unit (11) which receives a query to a database from a user, creates a user query for searching information from a database in accordance with the query of the user, and outputs the created user query; a query generating unit (12) which receives the user query or a re-search query for re-searching information from a database, generates a database query for actually searching information from a database in accordance with the user query or the re-search query, and outputs the created database query; a searching unit (13) which executes a search of information in accordance with the database query, and outputs an information searching result; an analyzing unit (14) which analyzes the information searching result output by the searching unit (13) and outputs an information analyzing result; an output control unit (15) which outputs the information analyzing result from the analyzing unit (14) as output information, creates the re-search query by using the information analyzing result, and supplies the created re-search query to said query generating unit (12); and a condition setting unit (16) which has an analyzing condition for analyzing the information searching result and an output condition for outputting the output information, supplies the analyzing condition to said analyzing unit (14), and supplies the output condition to said output control unit (15). The analyzing unit (14) analyzes the information searching result in accordance with the analyzing condition from the condition setting unit (16); and the output control unit (15) outputs the output information in accordance with the output condition from said condition setting unit (16).

The information search system may further include an output setting unit (21) which receives conditions, concerning an analyzing of the information searching result and an output of the output information, from the user, and provides the received conditions to said condition setting unit (16). The condition setting unit (16) may hold the conditions from said output setting unit (21) as the analyzing condition and the output condition.

The information search system may further include a document searching unit (51) which receives the output information output by the output control unit (15), searches a document related to the output information from database, and outputs a document searching result.

The information search system may further include a second analyzing unit (61) which receives the document searching result, analyzes the document searching result by extracting predetermined information from the document searching result, and outputs a document analyzing result.

The information search system may further include a format changing unit (71) which receives the document analyzing result, changes a format of the document analyzing result into a predetermined format, and outputs a document analyzing result whose format is changed.

The information search system may further include a complementing unit (41) which receives the output information output by said output control unit (15), and complements the output information with information concerning the output information.

The information to be searched may be biological information.

An information search system according to a second aspect of the present invention includes a second input receiving unit (22) which receives a query to a database and a designation of information to be output, from a user, creates a user query for searching information from a database and a designation information representing the information to be output, in accordance with the query and the designation of the user, and outputs the user query and the designation information; a search procedure setting unit (32) which sets a search procedure in accordance with the user query and the designation information, and outputs a database query list representing the search procedure; a search control unit (33) which controls a search of information from the database and an analyzing of an information searching result, in accordance with the database query list and the user query, and outputs an information analyzing result as output information; a searching unit (13) which executes the search of information under control of said search control unit (33), and outputs the information searching result; an analyzing unit (14) which executes the analyzing of the information searching result under control of said search control unit (33), and outputs the information analyzing result to the search control unit (33); a condition setting unit (16) which has an analyzing condition for analyzing the information searching result, and supplies the analyzing condition to said analyzing unit (14). The analyzing unit (14) executes the analyzing of the information searching result in accordance with the analyzing condition from the condition setting unit (16).

The information search system may further include a document searching unit (51) which receives the output information output by said search control unit (33), searches a document related to the output information from a database, and outputs a document searching result.

The information search system may further include a second analyzing unit (61) which receives the document searching result, analyzes the document searching result by extracting predetermined information from the document searching result, and outputs a document analyzing result.

The information search system may further include a format changing unit (71) which receives the document analyzing result, changes a format of the document analyzing result into a predetermined format, and outputs a document analyzing result whose format is changed.

The information search system may further include a complementing unit (41) which receives the output information output by the search control unit (33), and complements the output information with information concerning the output information to the output information.

The information to be searched is biological information.

An information search method according to a third aspect of the present invention includes the steps of receiving a query to a database from a user; creating a user query for searching information from a database in accordance with the query of the user; creating a database query for actually searching information from a database in accordance with the user query or a re-search query for re-searching information from a database; executing a search of information in accordance with the database query; analyzing an information searching result obtained by the search of information, in accordance with an analyzing condition for analyzing the information searching result; creating the re-search query by using an information analyzing result which is obtained by analyzing the information searching result; outputting the information analyzing result as output information, in accordance with an output condition for outputting the information analyzing result.

The information search method may further include the steps of receiving conditions concerning an analyzing of the information searching result and an output of the output information, from the user; and holding the conditions as the analyzing condition and the output condition.

The outputting the output information may include outputting the output information every time when the analyzing of the information searching result is executed.

The outputting the output information may include holding the information analyzing result which is obtained by each analyzing of the information searching result; and outputting the held information analyzing results in one time, in a case where all re-searches are completed.

The information search method may further include the steps of searching a document related to the output information from a document; and outputting a document searching result obtained by the searching.

The information search method may further include the steps of analyzing the document searching result by extracting predetermined information from the document searching result; outputting a document analyzing result obtained by analyzing the document searching result.

The information search method may further include the steps of changing a format of the document analyzing result into a predetermined format; and outputting a document analyzing result whose format is changed.

The information search method may further include the step of complementing the output information with information concerning the output information.

The information to be searched may be biological information.

An information search method according to a fourth aspect of the present invention includes the step of receiving a query to a database and a designation of information to be output, from a user; creating a user query for searching information from a database and a designation information representing the information to be output, in accordance with the query and the designation of the user; setting a search procedure in accordance with the user query and the designation information, thereby a database query list representing the search procedure is created; creating a database query for controlling a search of information from the database, in accordance with the database query list and the user query; executing the search of information in accordance with the database query; analyzing an information searching result obtained by executing the search, in accordance with an analyzing condition for analyzing the information searching result; and outputting an information analyzing result obtained by analyzing the information searching result, as output information, in accordance with an output condition for outputting the output information.

The information search method may further include the steps of searching a document related to the output information from a database; and outputting a document searching result obtained by the searching.

The information search method may further include the steps of analyzing the document searching result by extracting predetermined information from the document searching result; outputting a document analyzing result obtained by analyzing the document searching result.

The information search method may further include the steps of changing a format of the document analyzing result into a predetermined format; and outputting a document analyzing result whose format is changed.

The information search method may further include the step of complementing the output information with information concerning the output information.

The information to be searched may be biological information.

These objects and other objects and advantages of the present invention will become more apparent upon reading of the following detailed description and the accompanying drawings in which:
FIG. 1 is a configuration view of a biological information search system according to a first embodiment;
FIG. 2 is a flowchart illustrating processing performed by a query generating section included in the system of FIG 1;
FIG. 3 is a flowchart illustrating processing performed by the query generating section;
FIG. 4 is a flowchart illustrating processing performed by a biological information search section included in the system of FIG 1;
FIG. 5 is a flowchart illustrating another processing performed by the biological information search section;
FIG. 6 is a flowchart illustrating processing performed by an analyzing section included in the system of FIG. 1;
FIG. 7 is a flowchart illustrating processing performed by an output control section included in the system of FIG. 1;
FIG. 8 is a flowchart illustrating another processing performed by an output control section;
FIG. 9 is a flowchart illustrating another processing performed by an output control section;
FIG. 10 is a flowchart illustrating processing performed by a condition setting section included in the system of FIG. 1;
FIG. 11 is a view illustrating an input form displayed by an input receiving section;
FIG. 12 is a view illustrating an example of a searching result;
FIG. 13 is a view illustrating an example of a searching result;
FIG. 14 is a view illustrating an example of a searching result;
FIG. 15 is a view illustrating an example of a searching result;
FIG. 16 is a configuration view of a biological information search system according to a second embodiment;
FIG. 17 is another configuration view of the biological information search system according to the second embodiment;
FIG. 18 is a view illustrating an input screen displayed by an outputting section included in a system of FIG. 16;
FIG. 19 is a configuration view of a biological information system according to a third embodiment;
FIG. 20 is a flowchart illustrating processing performed by a search procedure setting section included in a system of FIG. 19;
FIG. 21 is a flowchart illustrating processing performed by a search control section 33 that configures the system of FIG. 19;
FIG. 22 is a configuration view of a biological information search system according to a fourth embodiment;
FIG. 23 is a configuration view of a biological information search system according to a fifth embodiment;
FIG. 24 is a flowchart illustrating processing performed by a document search section included in a system of FIG. 23;
FIG. 25 is a view illustrating one example of a searching result;
FIG. 26 is a view illustrating one example of a searching result;
FIG. 27 is a configuration view of a biological information search system according to a sixth embodiment;
FIG. 28 is a view illustrating one example of a searching result;
FIG. 29 is a view illustrating one example of a searching result;
FIG. 30 is a view illustrating one example of a result generated by a second analyzing section included in a system of FIG. 27;
FIG. 31 is a configuration view of a biological information search system according to a seventh embodiment; and
FIG. 32 is a view illustrating one example of a result output by a form changing section included in a system of FIG. 31.

The following will explain a biological information search system and biological information search method according to a first embodiment of the present invention with reference to the drawings.

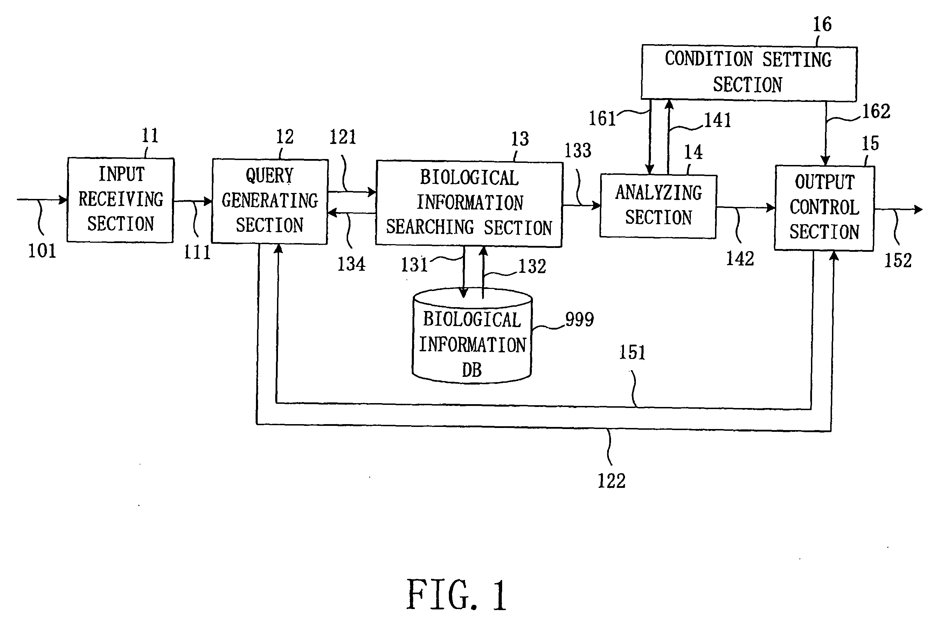

FIG. 1 is a configuration view of a biological information search system according to the first embodiment. As illustrated in FIG. 1, the biological information search system includes an input receiving section 11, a query generating section 12, a biological information searching section 13, an analyzing section 14, an output control section 15, and a condition setting section 16.

The biological information search system is realized by a computer including an input device with a keyboard and a mouse, a display with a CRT (Cathode Ray Tube) or a liquid crystal panel, a memory, and a CPU (Central Processing Unit). More specifically, the memory stores a program and data for performing processing to be described later, and the CPU executes the program stored in the memory. This realizes the biological information search system having the configuration of FIG. 1.

The biological information search system is connected to multiple DB (Data Base) servers each having a biological information DB 999 via a network such as the Internet and the like. The biological information search system obtains information relating to information input by a user from the multiple biological information DBs 999 as described later. Additionally, in FIG. 1, only one biological information DB 999 is illustrated as an example.

The input receiving section 11 receives information (user input) 101 that the user inputs by use of the input device. The user input 101 includes at least one keyword for searching biological information from the biological information DB 999. The keyword includes, for example, a gene or protein name, an accession number, or a base or amino acid sequence.

The input receiving section 11 specifies a kind of each keyword included in the received user input 101. The input receiving section 11 brings each keyword into correspondence with information indicating the specified kind to generate a user query 111. The user query 111 shows a list of combinations {keyword, kind of keyword). The input receiving section 11 supplies the generated user query 111 to the query generating section 12.

The following two methods can be used as a method for specifying the keyword and the kind of keyword.

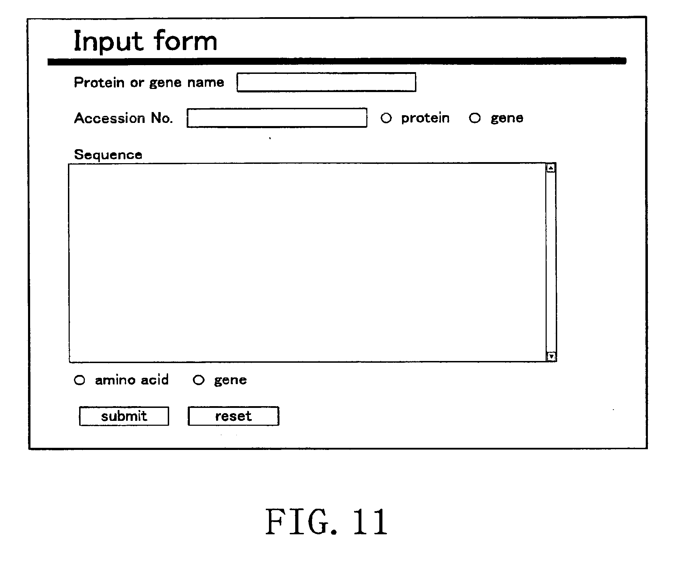

A first method is one that uses an input form for inputting a keyword. For example, the input receiving section 11 has data of an input form as illustrated in FIG. 11. The input form is provided with an input field for each kind of keyword. The input receiving section 11 displays the input form on the display using data. The user inputs a keyword in at least one input field using the input device. The input receiving section 11 specifies the keyword and the kind of keyword depending on in which input field the user inputs the keyword.

A second method is one that uses a format of a keyword. For example, the input receiving section 11 has format data of a gene or protein name, an accession number, base and amino acid names, and base and amino acid sequences. The input receiving section 11 specifies a character string that complies with the format, from the user input 101 using format data. The input receiving section 11 thereby specifies the keyword and the kind of keyword.

Additionally, when the kind of keyword is not specified to one, the input receiving section 11 brings the keyword into correspondence with each of all kinds that the keyword can take.

The query generating section 12 has biological DB information. The biological DB information shows a location of each biological information DB 999 (DB location), a kind of usable keyword in each biological information DB 999. Namely, the biological DB information shows a list of combinations {DB location, kind of usable keyword}. In addition, the location of biological information DB 999 can be expressed by, for example, a URL (Uniform Resource Locator).

The query generating section 12 further includes data of a flag table having search flags each corresponding to a combination included in the biological DB information. Each search flag shows whether a search corresponding to each combination included in the biological DB information is conducted.

The query generating section 12 initializes the flag table in response to the user query 111 from the input receiving section 11. As a result, all search flags are set to a state (value) indicating "unsearched."

The query generating section 12 compares each kind of keyword shown by the user query 111 with each kind of usable keyword shown by the biological DB information. The query generating section 12 generates a DB query 121 including information necessary for searching by use of the combination of the user query 111 and the combination of biological DB information each indicating the same kind. The DB query 121 shows a list of combinations {DB location, keyword, kind of keyword). Accordingly, a biological DB 999 used in searching is decided.

The query generating section 12 supplies the generated DB query 121 to the biological information searching section 13. At this time, the query generating section 12 changes the search flag corresponding to each combination of the generated DB query 121 from "unsearched" to "searched."

The biological information searching section 13 generates an query signal 131 corresponding to each combination of DB query 121 by use of the DB query 121 supplied from the query generating section 12. Each query signal 131 includes the keyword shown by each combination of the DB query 121. The biological information searching section 13 transmits the corresponding query signal 131 to the DB location shown by each combination of the supplied DB query 121. As a result, each DB server that received the query signal 131 conducts a search.

The DB server that received the query signal 131 searches biological information relating to the keyword from the biological information DB 999 that the DB server includes, by use of the keyword shown by the query signal 131. The DB server transmits a searching result 132 to the biological information searching section 13.

The biological information searching section 13 adds information (DB specifying information) for specifying the used biological information DB 999 and information indicating the used keyword and the kind of the used keyword to the searching result 132 supplied from each DB server. As a result, the biological information searching section 13 generates a DB searching result 133. The DB searching result 133 shows the searching result 132, the DB specifying information, the keyword, and the kind of keyword. The DB specifying information is, for example, a name of biological information DB 999. The biological information searching section 13 supplies the generated DB searching result 133 to the analyzing section 14.

Additionally, when an error occurs in the DB server, the DB server transmits the searching result 132 showing occurrence of the error to the biological information searching section 13. In this case, the biological information searching section 13 supplies error information 134 indicating occurrence of the error to the query generating section 12. Moreover, when the searching result 132 cannot be obtained within a predetermined standby time, the biological information searching section 13 determines that an error such as communication trouble and the like occurs. In this case, the biological information searching section 13 also supplies error information 134 to the query generating section 12. The query generating section 12 returns the corresponding search flag to "unsearched" in response to the error information 134 from the biological information searching section 13.

Further, the biological information searching section 13 may transmit the same query signal 131 for each standby time, and when the number of transmissions reaches the predetermined number of times, the error information 134 may be supplied. In this case, the number of transmissions is preset to the biological information searching section 13.

The analyzing section 14 generates a condition request 141 using the DB searching result 133 from the biological information searching section 13. The condition request 141 indicates the presence or absence of error, DB specifying information, and the kind of keyword. The analyzing section 14 supplies the generated condition request 141 to the condition setting section 16.

The condition setting section 16 prepares data of an extraction condition 161 according to a combination of the used biological information DB 999, the kind of the used keyword and the presence or absence of the error, in advance. The extraction condition 161 shows a condition for extracting information to be output from the DB searching result 133. More specifically, the extraction condition 161 shows a format of the searching result 132, a part where information to be extracted is described, a rule for extracting information, and a format of the searching result 132 when an error occurs. The condition setting section 16 supplies the extraction condition 161 corresponding to the condition request 141 to the analyzing section 14 in response to the condition request 141 from the analyzing section 14.

The analyzing section 14 extracts information from the DB searching result 133 according to the extraction condition 161 from the condition setting section 16. Information to be extracted is, for example, DB specifying information, the keyword, the kind of keyword, and biological information (names and bynames of the gene and protein, the base and amino acid sequences, accession number, etc) relating to the keyword. The analyzing section 14 supplies information extracted from the DB searching result 133 to the output control section 15 as an analyzing result 142.

The output control section 15 provides a flag set according to an output condition 162 from the condition setting section 16. The output condition 162 shows biological information requested as a result obtained by searching. The biological information requested as a result includes, for example, the gene or protein name obtained from Entrez and the gene or protein name obtained from LocusLink. The flag set includes output flags each corresponding to the biological information to be requested. Each output flag shows whether the requesting biological information is obtained.

The output control section 15 holds the analyzing result 142 supplied from the analyzing section 14. The output control section 15 detects the requesting biological information corresponding to the output flag showing "non-obtained", from the analyzing result 142. The output control section 15 changes the output flag corresponding to the detected biological information to a state of "obtained." Accordingly, when all output flags are in a state showing "obtained", the output control section 15 outputs all held analyzing results 142 as output information 152 at one time. At this time, the output control section 15 controls the display and the like, so that the list of information shown by all held searching results 142 may be displayed. As a result, the search is completed. Additionally, when the search is completed, the output control section 152 controls the display and the like, so that information indicating the completion of search may be output. This makes it possible for the user to clearly show the completion of search.

On the other hand, in a case where the output flag showing "non-obtained" is left, the output control section 15 extracts biological information and information indicating the kind from the held analyzing result 142. The output control section 15 brings each extracted biological information into correspondence with information indicating the kind, so that a re-search query 151 is generated. The re-search query 151 shows a list of combinations {biological information, kind of biological information}. The biological information shown by the re-search query 151 is used as a keyword for re-search. The output control section 15 supplies the generated re-search query 151 to the query generating section 12. As a result, a re-search is conducted.

In addition, the output control section 15 may the analyzing results 142, sequentially. In this case, the output control section 15 outputs the analyzing result 142 as output information 152, upon reception of the analyzing result 142. In a case where the output flag showing "non-obtained" is left, the output control section 15 supplies the re-search query 151 to the query generating section 12, similar to the above.

After supplying the re-search query 151, processing performed by the query generating section 12 is the same as the above. Moreover, at the re-searching time, processing performed by each of the biological information searching section 13, the analyzing section 14 and the output control section 15 is the same as the above. However, the query generating section 12 does not initialize the flag table when re-search query 151 is supplied thereto. Moreover, in a case where all kinds of keywords shown by the re-search query 151 are already searched, the query generating section 12 outputs search completion information 122 indicating the completion of search to the output control section 15. The output control section 15 outputs all held analyzing results 142 as output information 152 at one time regardless of the output flag state when the search completion information 122 is supplied thereto. At this time, the output control section 15 may output information indicating the completion of search. As a result, the search is completed.

The condition setting section 16 includes data of the aforementioned extraction condition 161 in advance. The condition setting section 16 supplies the extraction condition 161 corresponding to the condition request 141 to the analyzing section 14, in response to the condition request 141 from the analyzing section 14. Moreover, the condition setting section 16 includes data of the aforementioned output condition 162 in advance. The condition setting section 16 supplies the output condition 162 to the output control section 15 in response to the first condition request 141 from the analyzing section 14. The output condition 162 includes the initial value of the aforementioned flag set.

The input receiving section 11 receives the user input 101 input by the user. The input receiving section 11 specifies a kind of each keyword included in the received user input 101. The input receiving section 11 brings each keyword included in the user input 101 into correspondence with information indicating the specified kind to generate a user query 111.

As explained above, as a method for specifying the keyword and the kind of keyword, there are two methods including one using the input form and the other using the keyword format.

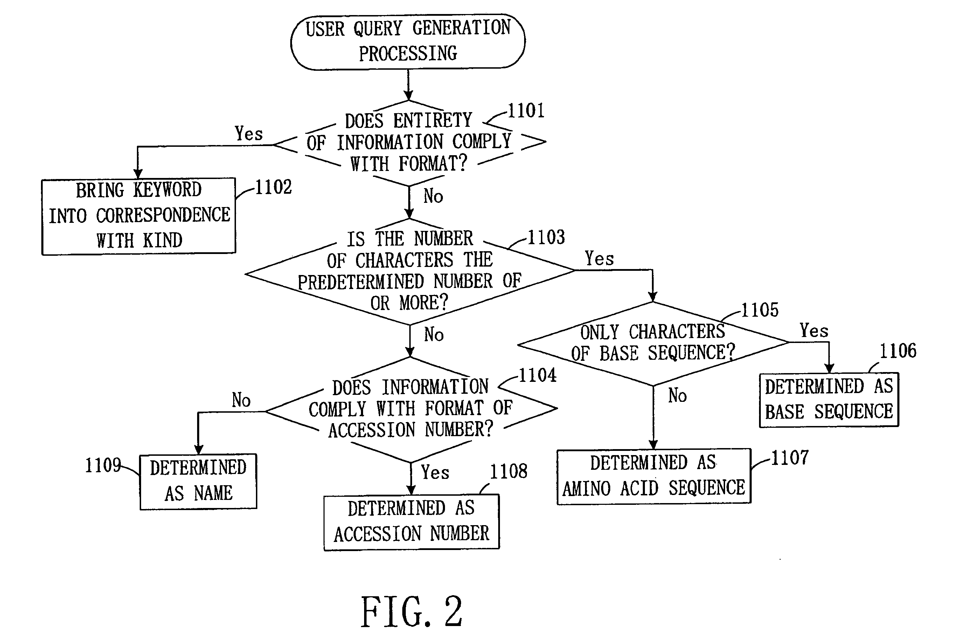

The following will explain operations of the input receiving section 11 when the keyword format is used with reference to FIG. 2.

In this case, the input receiving section 11 has format data of names of a gene and a protein, an accession number, base and amino acid names, and base and amino acid sequences. The format may be one that is provided in public and well known and used in, for example, Entrez, LocusLink, and SWISS-PROT.

First of all, the input receiving section 11 determines whether the entirety of information indicated by the user input 101 is described according to the format, by using format data (step 1101).

For example, a FASTA format is one that is well known among the profession. According to this format, ">" is described at the top of the first line, and a gene or protein name is described subsequent thereto. Then, at the second line and afterward, a base or amino acid sequence is described. A GenBank format is also well known among the profession. In this format, the formats of such as a gene name, a protein name, a base sequence, an amino acid sequence, a species, a published scholarly paper, etc are standardized.

In a case where the input receiving section 11 determines that the entirety of information is described according to the format (step 1101: Yes), the input receiving section 11 specifies one or multiple keywords included in the user input 101 and the kind of the specified one or multiple keywords, by use of the format data. The input receiving section 11 extracts the specified keyword from the user input 101 and brings the extracted keyword into correspondence with the specified kind (step 1102).

On the other hand, in a case where the input receiving section 11 does not determine that the entirety of information is described according to the format (step 1101: No), the input receiving section 11 obtains the number of characters of information shown by the user input 101. The input receiving section 11 determines whether the obtained number of characters is the predetermined number of characters (for example, 20 characters) or more (step 1103).

In a case where the input receiving section 11 determines that the obtained number of characters is the predetermined number of characters or more (step 1103; Yes), the input receiving section 11 determines that the user input 101 shows the sequence. Then, the input receiving section 11 specifies the kind of the sequence shown by the user input 101. More specifically, the input receiving section 11 determines whether the user input 101 includes only the characters used in the base sequence (step 1105). The characters used in the base sequence are G, A, T, G (or X, N in addition to C, A, T, C).

In a case where the input receiving section 11 determines that the user input 101 includes only the characters used in the base sequence (step 1105; Yes), the input receiving section 11 determines that the user input 101 shows the base sequence (step 1106). The input receiving section 11 brings the keyword shown by the user input 101 into correspondence with the kind ''base sequence."

On the other hand, in a case where the input receiving section 11 determines that the user input 101 includes the characters except those used in the base sequence (step 1105; No), the input receiving section 11 determines that the user input 101 shows the amino acid sequence (step 1107). The input receiving section 11 brings the keyword shown by the user input 101 into correspondence with the kind "amino acid sequence."

In a case where the input receiving section 11 determines that the obtained number of characters is less than the predetermined number of characters (step 1103; No), the input receiving section 11 determines that the user input 101 does not show the sequence. The input receiving section 11 determines whether information indicated by the user input 101 complies with the format (combination of alphanumeric characters) of the accession number (step 1104).

In a case where the input receiving section 11 determines that information complies with the format of the accession number (step 1104; Yes), the input receiving section 11 determines that the user input 101 shows the accession number (step 1108). The input receiving section 11 brings the keyword shown by the user input 101 into correspondence with the kind "accession number."

On the other hand, in a case where the input receiving section 11 does not determine that information complies with the format of the accession number (step 1104; No), the input receiving section 11 determines that the user input 101 shows the name (step 1109). The input receiving section 11 brings the keyword shown by the user input 101 into correspondence with the kind "name."

By the aforementioned processing, a user query 111 indicating the list of the combinations {keyword, kind of keyword} is generated. The input receiving section 11 supplies the generated user query 111 to the query generating section 112.

In the aforementioned processing, if the kind of keyword is not specified to one, the input receiving section 11 brings the keyword into correspondence with each of all kinds that the keyword can take.

Next, the following will specifically explain processing performed by the query generating section 12.

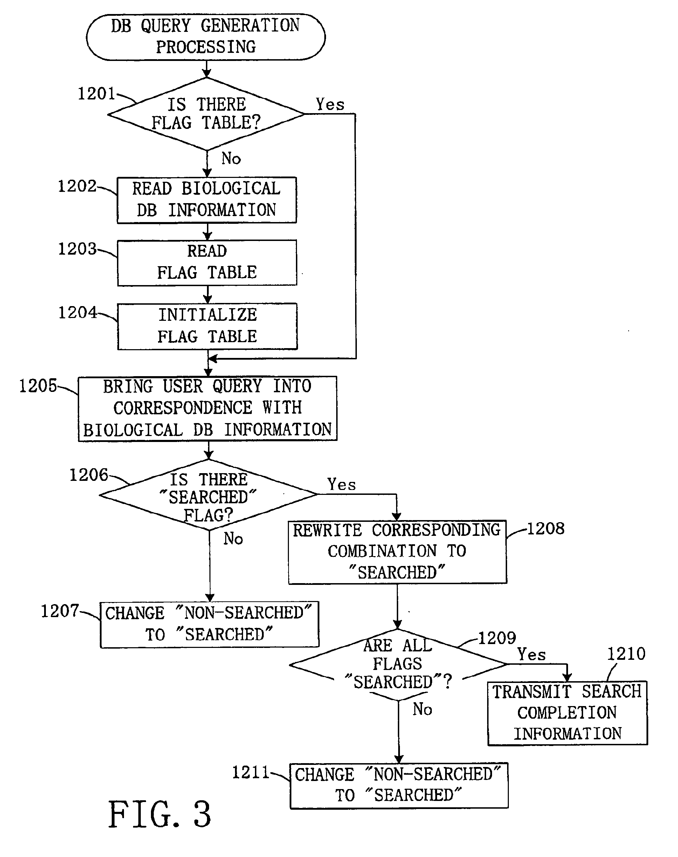

The user query 111 is supplied to the query generating section 12 from the input receiving section 11 and the re-search query 151 is supplied thereto from the output control section 15. The query generating section 12 starts processing illustrated in FIG. 3 in response to the supplied user query 111 or re-search query 151.

First of all, the query generating section 12 determines whether there is a flag table in use (step 1201).

In a case where the query generating section 12 determines that there is a flag table in use (step 1201; Yes), the query generating section 12 proceeds to step 1205 to be described later. Additionally, the presence of the flag table in use means that the query generating section 12 receives the re-search query 151.

On the other hand, in a case where the query generating section 12 determines that there is no flag table in use (step 1201; No), the query generating section 12 reads biological DB information (step 1202). Additionally, the absence of the flag table in use means that the query generating section 12 receives the user query 111.

The query generating section 12 reads data of the flag table (step 1203). As a result, there are provided search flags each corresponding to the combination {DB location, kind of usable keyword} included in the biological DB information.

The query generating section 12 initializes the read flag table to set all search flags to the state showing "non-search" (step 1204).

The query generating section 12 compares each kind of keyword shown by the user query 111 (or re-search query 151) with each kind of usable keyword shown by the biological DB information. The query generating section 12 brings each combination of the user query 111 indicating the same kind into correspondence with each combination of the biological DB information (step 1205). As a result, a DB query 121 is generated. The DB query 121 shows the list of combinations {DB location, keyword, kind of keyword}. In addition, if there is no biological information DB 999 where the keyword shown by the user query 111 can be used, the query generating section 12 brings information indicating search disability into correspondence with a keyword and its kind.

After that, the query generating section 12 determines whether there is a search flag showing "searched" among the flags each corresponding to each combination of the generated DB query 121 (step 1206).

In a case where the query generating section 12 determines that there is no search flag showing "searched" (step 1206; No), the query generating section 12 changes the search flags each corresponding to each combination of the generated DB query 121 from "non-searched" to "searched" (step 1207). Then, the query generating section 12 supplies the DB query 121 to the biological information searching section 13.

On the other hand, in a case where the query generating section 12 determines that there is a search flag showing "searched" (step 1206; Yes), the query generating section 12 rewrites the contents of the combination corresponding to the search flag, to information indicating "searched" (step 1208).

Sequentially, the query generating section 12 determines whether all search flags corresponding to the combinations included in the DB query 121 show "searched" (step 1209).

In a case where the query generating section 12 determines that all corresponding search flags show "searched" (step 1209; Yes), the query generating section 12 supplies search completion information 122 indicating the completion of search to the output control section 15 (step 1210).

On the other hand, in a case where the query generating section 12 does not determine that all corresponding search flags show "searched" (step 1209; No), the query generating section 12 changes the corresponding search flags showing "non-searched" to "searched" (step 1211). Then, the query generating section 12 supplies the DB query 121 to the biological information searching section 13.

Next, the following will specifically explain processing performed by the biological information searching section 13.

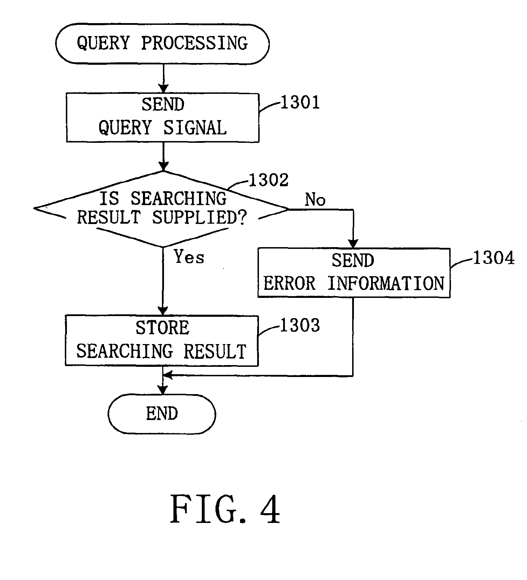

The biological information searching section 13 starts processing illustrated in FIG 4 in response to the DB query 121 supplied from the query generating section 12. In addition, the biological information searching section 13 performs processing of FIG 4 to each combination of the DB query 121.

First of all, the biological information searching section 13 obtains one combination from the supplied DB query 121. The biological information searching section 13 generates a query signal 131 including the keyword shown by the obtained combination. The biological information searching section 13 transmits the generated query signal 131 to the DB location shown by the obtained combination (step 1301). As a result, the DB server that received the query signal 131 starts a search by use of the biological information DB 999.

The biological information searching section 13 waits for the supply of the searching result 132 from the DB server. The biological information searching section 13 determines whether the searching result 132 is supplied from the DB server within a predetermined standby time (step 1302).

In a case where the biological information searching section 13 determines that the searching result 132 is supplied (step 1302; Yes), the biological information searching section 13 stores the supplied the searching result 132 (step 1303). In addition, in a case where an error occurs in the DB server, the searching result 132 shows the occurrence of error in the DB server.

On the other hand, in a case where the biological information searching section 13 does not determine that the searching result 132 is supplied within a predetermined standby time (step 1302; No), the biological information searching section 13 determines that an error such as communication trouble and the like occurs. The biological information searching section 13 supplies error information 134 to the query generating section 12 (step 1304). The query generating section 12 returns the corresponding search flag to "non-searched" in response to the error information 134 from the biological information searching section 13.

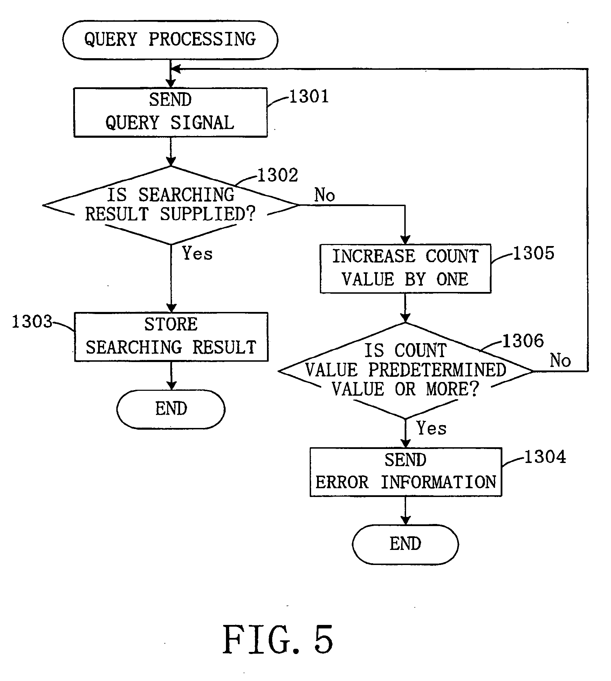

As mentioned above, in a case where the biological information searching section 13 does not determine that the searching result 132 is supplied within a predetermined standby time, the biological information searching section 13 may transmit the same query signal 131 to the same DB server. In this case, the biological information searching section 13 includes a counter that counts the number of transmissions of query signal 131 to perform processing according to the flow of FIG 5.

In a case where the biological information searching section 13 does not determine that the searching result 132 is supplied within the predetermined standby time (step 1302; No), the biological information searching section 13 increases a counter value by one (step 1305).

The biological information searching section 13 determines whether the counter value is a predetermined value or more (step 1306).

In a case where the biological information searching section 13 determines that the counter value is below the predetermined value (step 1306; No), the biological information searching section 13 goes back to step 1301. The biological information searching section 13 retransmits the query signal 131 and tries to conduct a re-search.

On the other hand, in a case where the biological information searching section 13 determines that the counter value is the predetermined value or more (step 1306; Yes), the biological information searching section 13 supplies error information 134 to the query generating section 12 (step 1304).

When receiving the searching result 132 from the DB server, the biological information searching section 13 adds DB specifying information and information, which indicates the used keyword and the kind of the used keyword, to the received searching result 132. As a result, the biological information searching section 13 generates a DB searching result 133. The biological information searching section 13 supplies the generated DB searching result 133 to the analyzing section 14.

Next, the following will specifically explain processing performed by the analyzing section 14.

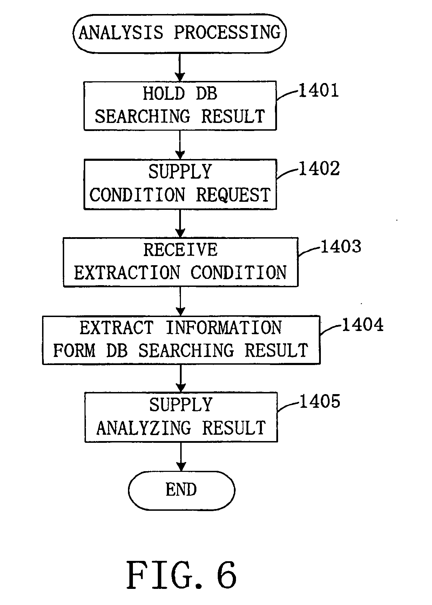

FIG 6 is a flowchart illustrating processing performed by the analyzing section 14.

The analyzing section 14 holds the DB searching result 133 supplied from the biological information searching section 13 (step 1401).

The analyzing section 14 generates a condition request 141 indicating the presence or absence of an error, DB specifying information, and the kind of keyword. The condition request 141 supplies the generated condition request 141 to the condition setting section 16 (step 1402). The condition setting section 16 supplies the extraction condition 161correspodning to the condition request 141 to the analyzing section 14 in response to the condition request 141 from the analyzing section 14.

The analyzing section 14 receives the extraction condition 161 supplied from the condition setting section 16(step 1403).

The analyzing section 14 extracts information from the DB searching result 133, according to the supplied extraction condition 161 (step 1404). Information to be extracted is, for example, DB specifying information, a keyword, the kind of keyword, and biological information (names and bynames of the gene and protein, the base and amino acid sequences, accession number, etc) relating to the keyword.

At this time, there is a case in which multiple information of the same kind is extracted. In this case, the analyzing section 14 may select one of information of the same kind. For example, when the gene or protein name is used as a keyword, there is a case in which accession numbers of multiple genes or protein, each having a different species or mutation or proteins, are obtained by searching. In this case, the multiple accession numbers are extracted from the DB searching result 133. The analyzing section 14 may select one accession number according to a predetermined rule. The predetermined rule includes, for example, the format of the name, the format of the accession number, and the sequence of information.

The analyzing section 14 supplies information as the analyzing result 142 extracted from the DB searching result 133 to the output control section 15 (step 1405).

Next, the following will specifically explain processing performed by the output control section 15.

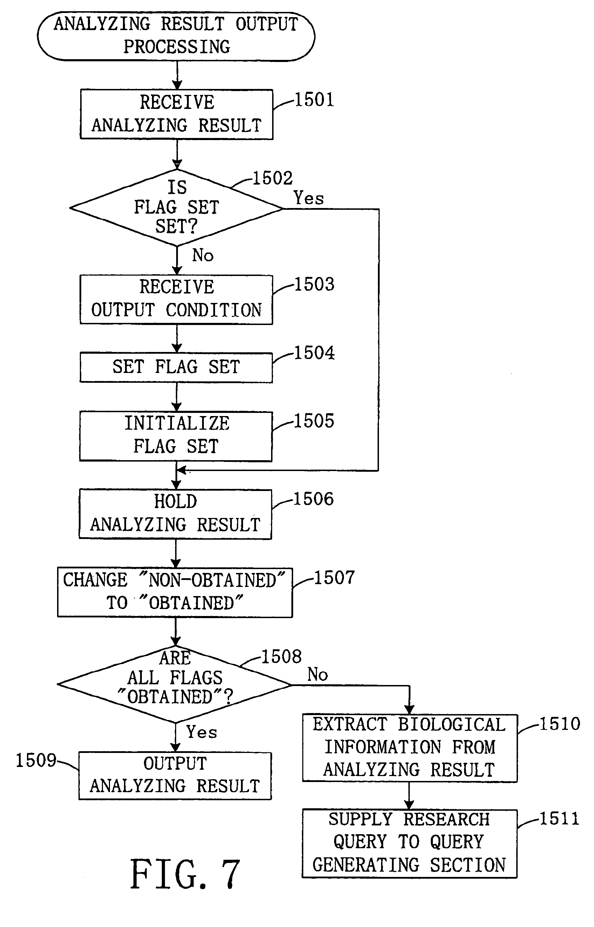

First of all, an explanation is given of the case in which the output control section 15 holds the analyzing result 142 from the analyzing section 14 and outputs all analyzing results 142 held at the time of the search completion at one time, with reference to FIG 7.

The output control section 15 receives the analyzing result 142 supplied from the analyzing section 14 (step 1501).

The output control section 15 determines whether the flag set is already provided (step 1502). In a case where the analyzing result 142 is obtained by re-searching, the flag set is already provided.

In a case where the output control section 15 determines that the flag set is already provided (step 1502; Yes), the output control section 15 proceeds to step 1506 to be described later.

On the other hand, in a case where the output control section 15 does not determine that the flag set is already provided (step 1502; No), the output control section 15 receives the output condition 162 supplied from the condition setting section 16 (step 1503).

The output control section 15 provides a flag set according to the received output condition 162 (step 1504). The supplied analyzing result 142 is output when the condition based on the flag set is satisfied.

The output control section 15 initializes the provided flag set (step 1505). Accordingly, all output flags, each corresponding to biological information requested as a result obtained by searching, are set to a state (value) indicating "non-obtained."

The output control section 15 holds the supplied analyzing result 142 (step 1506).

The output control section 15 detects biological information corresponding to the output flag showing "non-obtained" from the analyzing result 142. The output control section 15 changes the output flag corresponding to the detected biological information to a state showing "obtained" (step 1507).

The output control section 15 determines whether all output flags show "obtained" (step 1508). As a result, the output control section 15 determines whether searching is completed.

In a case where the output control section 15 determines that all output flags show "obtained" (step 1508; Yes), the output control section 15 outputs all held analyzing results 142 as output information 152 at one time (step 1509).

On the other hand, in a case where the output control section 15 does not determine that all output flags show "obtained" (step 1508; No), the output control section 15 extracts biological information and information indicating the kind from the held analyzing result 142 (step 1510).

The output control section 15 brings the extracted biological information into correspondence with information indicating the kind to generate a re-search query 151. The re-search query 151 shows the list of the combinations {biological information, kind of biological information}. The output control section 15 supplies the generated re-search query 151 to the query generating section 12 (step 1511).

The biological information indicated by the re-search query 151 is used as a keyword for re-search. Additionally, in a case where multiple biological information of the same kind is extracted in step 1510, multiple biological information is combined by an OR condition, so that a keyword for re-search may be generated.

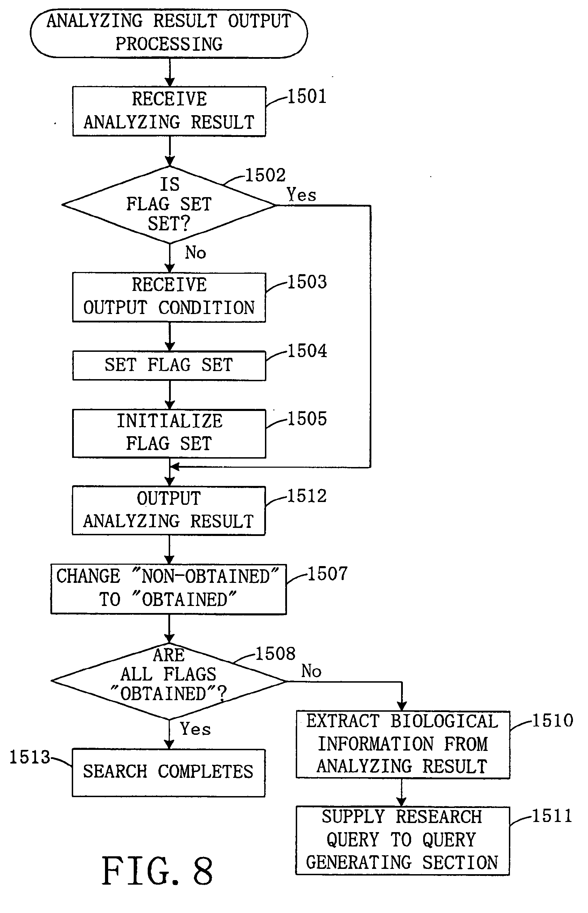

An explanation will be next given of the case in which every time when the output control section 15 receives the analyzing result 142 from the analyzing section 14, the output control section 15 outputs the received analyzing result 142 as output information 152, with reference to FIG 8.

In this case, processing except the steps 1512 and 1513 is the same as those of FIG 7. For this reason, the following explanation is given to mainly the steps 1512 and 1513.

After the output control section 15 receives the analyzing result 142 and provides the flag set, the output control section 15 outputs the received analyzing result 142 as output information 152 (step 1512).

After changing the corresponding output flags to the state showing "obtained", the output control section 15 determines whether all output flags show "obtained" (step 1508).

In a case where the output control section 15 does not determine that all output flags show "obtained" (step 1508; No), the output control section 15 performs processing in the aforementioned step 1510 and 1511. As a result, a re-search is performed.

On the other hand, in a case where output control section 15 determines that all output flags show "obtained" (step 1508; Yes), the output control section 15 ends searching directly (step 1513).

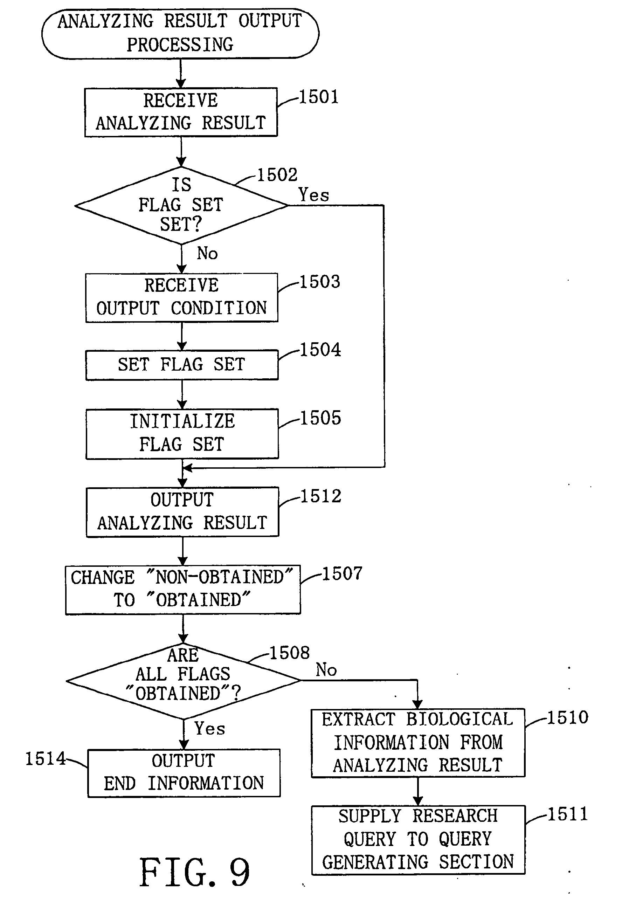

In addition, the output control section 15 may execute processing in step 1514 that outputs information indicating the completion of search in place of step 1513 as illustrated in FIG 9. Accordingly, it is possible to clarify that the user has completed the searching.

A detailed explanation will be next given of processing performed by the condition setting section 16.

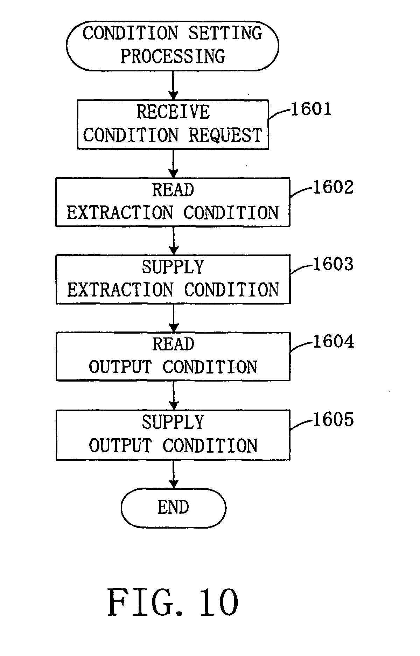

FIG 10 is a flowchart illustrating processing performed by the condition setting section 16.

The condition setting section 16 receives the condition request 141 supplied from the analyzing section 14 (step 1601).

The condition setting section 16 reads data of the prepared extraction condition 161 (step 1602).

The condition setting section 16 supplies the extraction condition 161 corresponding to the supplied condition request 141 to the analyzing section 14 (step 1603).

Sequentially, the condition setting section 16 reads data of the prepared output condition 162 (step 1604).

The condition setting section 16 supplies the output condition 162 to the output control section 15 (step 1605).

In addition, processing in step 1604 and 1605 is performed in response to the first condition request 141 from the analyzing section 14. Namely, such processing is not performed at the re-searching time.

An explanation will be next given of a specific example of operations of the biological information search system according to the first embodiment.

In the specific example, the user inputs a gene or protein name "pendrin." A name (byname) relating to "pendrin" is obtained from LocusLink by a fist search. At the same time, a GI number relating to "pendrin" is obtained from Entrez. At a second searching time, a base or amino acid sequence relating to the GI number is obtained. At a third search, a gene or protein having a high homology relating to the sequence is obtained from BLAST.

The input receiving section 11 displays an input form illustrated in, for example, FIG. 11 on the display. The input form has an input field for each kind of keyword. This makes it possible to designate the gene or protein name, the accession number, the base or amino acid sequence as keywords.

The user inputs "pendrin" in a filed of "Protein or gene name" and clicks a "Submit" button, by use of the input device. As a result, the input receiving section 11 receives the user input 101 including information input by user.

The input receiving section 11 specifies that "pendrin" is the gene or protein name based on information of the input field where the keyword is input. The input receiving section 11 brings the specified keyword "pendrin" into correspondence with the kind "gene or protein name" to generate a user query 111. The input receiving section 11 supplies the generated user query 111 to the query generating section 12.

As mentioned above, the keyword and the kind of keyword can be specified by the format of the keyword.

The query generating section 12 determines whether there is a flag table in use in response to the user query 111 from the input receiving section 11. At a first searching time, there is no flag table. For this reason, the query generating section 12 reads biological DB information, and sequentially reads data of the flag table. The query generating section 12 initializes the read flag table.

In this example, biological DB information indicates the following combinations.
Entrez 1 = {Entrez location 1, gene or protein name}
Entrez 2 = {Entrez location 2, GI number}
LocusLink 1 = {LocusLink location 1, gene or protein name}
BLAST 1 = {BLAST location 1, sequence information}
"Entrez location 1" is generated by combining a template of a search character string with URL of Entrez using CGI (Common Gateway Interface). A variable is set to a portion corresponding to the keyword of the template. The variable can be replaced with the keyword shown by the user query 111. The same can be applied to "Entrez location 2", "LocusLink location 1", and "BLAST location 1." URL may show the location of the local database.

In this embodiment, the search flags shown in below are provided. These search flags correspond to the combinations of biological DB information.
Entrez1 flag = {Entrez1 FALSE}
Entrez1 flag = {Entrez2, FALSE}
LocuLink1 flag = {LocusLink, FALSE}
BLAST1 flag = {BLAST1, FALSE}

When the search flag shows "non-searched", the state (value) of the search flag is expressed by FALSE. When the search flag shows "searched", the state (value) of the search flag is expressed by TRUE. The flag table is initialized to set the state of all search flags to FALSE.

The query generating section 12 brings each combination of the user query 111 showing the same kind into correspondence with each combination of biological DB information to generate a DB query 121.

In this case, the user query 111 shows the gene or protein name "pendrin." For this reason, the DB query 121, which shows the combinations {Entrez location 1, pendrin, gene or protein name} and {LocusLink location 1, pendrin, gene or protein name}, is generated.

Additionally, in this embodiment, the DB query 121 is generated by combining a search character string with URLs of Entrez and LocusLink using CGI. At this time, a variable provided in the template of the search character string can be replaced with "pendrin."

The query generating section 12 changes the search flags each corresponding to each combination of the generated DB query 121 from "non-searched" to "searched." In this case, the query generating section 12 changes the Entrez1 flag and LocusLink1 flag to {Entrez1, TRUE} and {LocusLink1, TRUE}, respectively. The query generating section 12 supplies the generated DB query 121 to the biological information searching section 13.

The biological information searching section 13 obtains a combination included in the DB query 121 from the query generating section 12. The biological information searching section 13 generates a query signal 131 including the keyword shown by each obtained combination. The biological information searching section 13 transmits each generated query signal 131 to the DB location shown by each combination. As a result, the first search is executed.

In this case, the biological information searching section 13 transmits the query signal 131 including "pendrin" as the keyword to Entrez and LocusLink, respectively. As a result, biological information relating to "pendrin" is searched from Entrez and LocusLink. The searching result 132 is supplied to the biological information searching section 13 from each of the DB server having Entrez and the DB server having LocusLink. In this example, the searching result 132 is supplied without occurrence of an error in each DB server.

The biological information searching section 13 stores the searching result 132 supplied from each DB server. Then, the biological information searching section 13 adds DB specifying information and information, which indicates the used keyword and the kind of the used keyword, to each searching result 132, so that a DB searching result 133 corresponding to each searching result 132 is generated. The biological information searching section 13 supplies the generated DB searching result to the analyzing section 14.

The analyzing section 14 supplies the condition request 141 to the condition setting section 16 in response to the DB searching result 133 from the biological information searching section 13. In this example, the analyzing section 14 supplies the condition request 141, which shows that no error occurs, Entrez is used, the kind of keyword is a gene or protein name, and the condition request 141, which shows that no error occurs, LocusLink is used, the kind of keyword is a gene or protein name.

The condition setting section 16 returns the extraction condition 161 corresponding to each condition request 141 in response to each supplied condition request 141. In this example, the condition setting section 16 supplies the extraction condition 161, which shows that a gene or protein name, an accession number, a GI number are extracted from the result obtained from Entrez, and the extraction condition 161, which shows that a byname of "pendrin" is extracted from the result obtained from LocusLink.

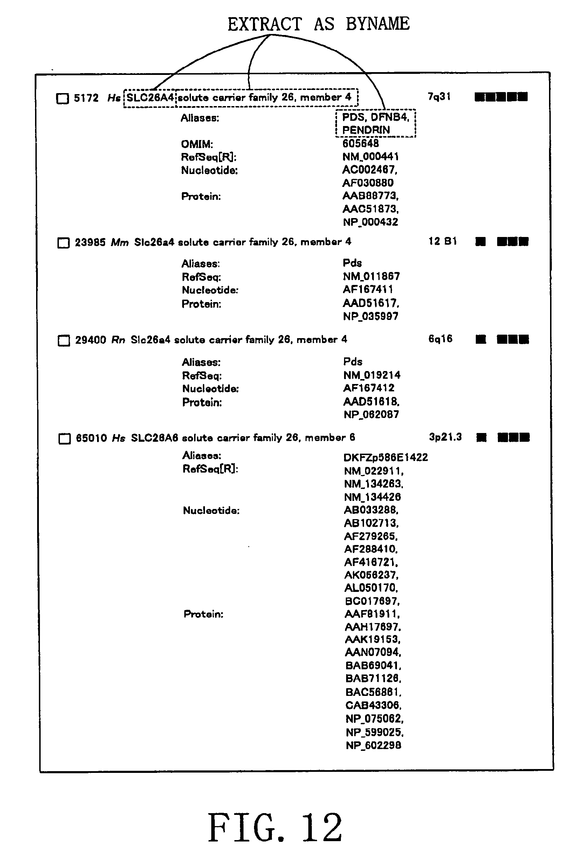

In the general search using Web browser, when biological information relating to "pendrin" is searched from LocusLink, the result as illustrated in FIG 12 can be obtained. As illustrated in FIG 12, in a predetermined portion of the obtained result, there are described an abbreviated name, a full name, an accession number used in the multiple biological information DB and a position of a gene on a chromosome. In addition, the multiple biological information DB is RefSeq, which is a database of genes or proteins where redundancy is excluded, and OMIM, which is a database of genes or proteins relating to diseases. Moreover, information of the gene or protein name is described in the fields of Symbol, Description, and Aliases of the obtained result.

Accordingly, in a case where the searching result 132 obtained from LocusLink is the same as that of FIG 12, information included in the predetermined portion of the result described in HTML (Hyper Text Markup Language) or information described in a predetermined format is extracted, thereby making it possible to obtain the gene or protein byname and the accession number. In this example, it is designated from the result obtained from LocusLink that information described in the fields of Symbol, Description, and Aliases is extracted.

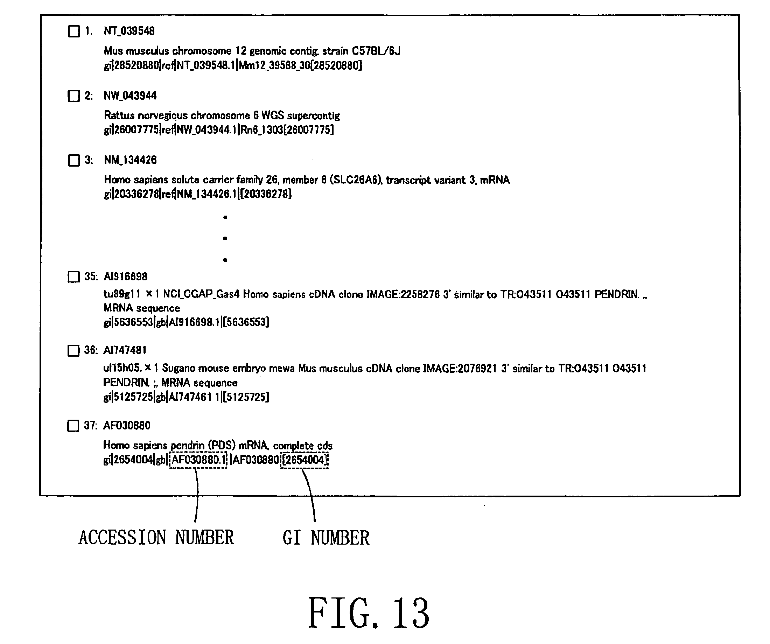

Furthermore, in the general search using Web browser, when biological information relating to "pendrin" is searched from Entrez, the result as illustrated in FIG 13 can be obtained. As illustrated in FIG 13, in a predetermined portion of the obtained result, there are described an accession number, a GI number, a gene or protein name used in GenBank or RefSeq.

Accordingly, similar to the case of LocusLink, information included in the predetermined portion of the result described in HTML (Hyper Text Markup Language) or information described in a predetermined format is extracted, thereby making it possible to obtain the gene or protein name, the accession number and the GI number. In this example, it is designated by the extraction condition 161 that information, which is described in such a format of "name mRNA, complete cds", and information, which is described in such a format of "| refINW#03954.8 |" that is the accession number used in RefSeq, are extracted. Furthermore, in order to obtain data of human, it is designated by the extraction condition 161 that a name "Homo sapiens" is included.

The analyzing section 14 extracts "PDS", "SLC26A4" and "DFNB4" as bynames for "pendrin" from the result obtained from LocusLink. The analyzing section 14 also extracts the name "Homo sapiens pendrin (PDS) mRNA, complete cds", the accession number "AF030880" and the GI number "2654004" from the result obtained from Entrez.

The analyzing section 14 supplies the analyzing result 142, which includes information extracted from the DB searching result 133 obtained from Entrez, and the analyzing result 142, which includes information extracted from the DB searching result 133 obtained from LocusLink, to the output control section 15.

In addition, the above showed the case in which data of human was designated as an example. However, data of other species may be, of course, designated.

When receiving the analyzing result 142 from the analyzing section 14, the output control section 15 determines whether the flag set is already provided. At a first searching time, the flag set is not provided. In this case, the output control section 15 provides a flag set according to the output condition 162 supplied from the condition setting section 16. The output control section 15 initializes the flag set to set all output flags to "non-obtained."

In this example, the following output flags are provided.
Output flag 1 = {Entrez, gene or protein name (byname), non-obtained}
Output flag 2 = {LocusLink gene or protein name (byname), non-obtained}
Output flag 3 = {BLAST, gene or protein name (byname), non-obtained}

The output control section 15 holds the supplied analyzing result 142. The output control section 15 detects biological information corresponding to the output flag, which shows "non-obtained", from each held analyzing result 142. The output control section 15 changes the output flag, which corresponds to the detected biological information, to a state showing "obtained." At a first searching time, a gene or protein byname is included in the analyzing result 142 from LocusLink. For this reason, the output control section 15 changes the output flag 2 from "non-obtained" to "obtained."

The output control section 15 determines whether all output flags show "obtained." At this point, the output flags 1 and 3 show "non-obtained." For this reason, the output control section 15 extracts a name "pendrin, SLC26A4, PDS and DFNB4", an accession number "AF03880", and a GI number "2654004" from the held analyzing result 142. The output control section 15 brings the extracted biological information into correspondence with the kind and supplies the result as a re-search query 151 to the query generating section 12.

The query generating section 12 determines whether there is a flag table in use, in response to the re-search query 151 from the output control section 15. At a second searching time, there is a flag table. For this reason, the query generating section 12 brings each combination of the re-search query 151, which shows the same kind, into correspondence with each combination of biological DB information, so that a DB query 121 is generated. In this case, the re-search query 151 shows the name "pendrin, SLC26A4, PDS and DFNB4", the accession number "AF03880", and the GI number "2654004." Accordingly, there is a search flag, which shows "searched", in the search flags corresponding to the combination included in the DB query 121.

The DB query 121 rewrites the contents of the combination, which corresponds to the search flag of "searched", to information indicating "searched." As a result, the DB query 121 shows a combination {Entrez location 2, 2654004, GI number}.

The query generating section 12 determines whether all search flags corresponding to the DB query 121 show "searched." In this case, Entrez2 flag shows "non-searched." Accordingly, the query generating section 12 changes Entrez2 flag from "non-searched" to "searched." After that, the query generating section 12 supplies the generated DB query 121 to the biological information searching section 13.

The biological information searching section 13 transmits the query signal 131 including the keyword to the DB location shown by DB query 121 supplied from the query generating section 12. In this case, the biological information searching section 13 transmits the query signal 131, which includes "2654004 (GI number)" to Entrez. As a result, biological information relating to "2654004 (GI number)" is searched from Entrez. The searching result 132 is supplied to the biological information searching section 13 from the DB server having Entrez.

The biological information searching section 13 stores the searching result 132 supplied from the DB server. The biological information searching section 13 generates a DB searching result 133 using the searching result 132, similar to the above. The biological information searching section 13 supplies the generated DB searching result 133 to the analyzing section 14.

The analyzing section 14 supplies the condition request 141 to the condition setting section 16 in response to the DB searching result 133 from the biological information searching section 13. At the second searching time, the analyzing section 14 supplies the condition request 141, which shows that no error occurs, Entrez is used, the kind of keyword is the GI number.

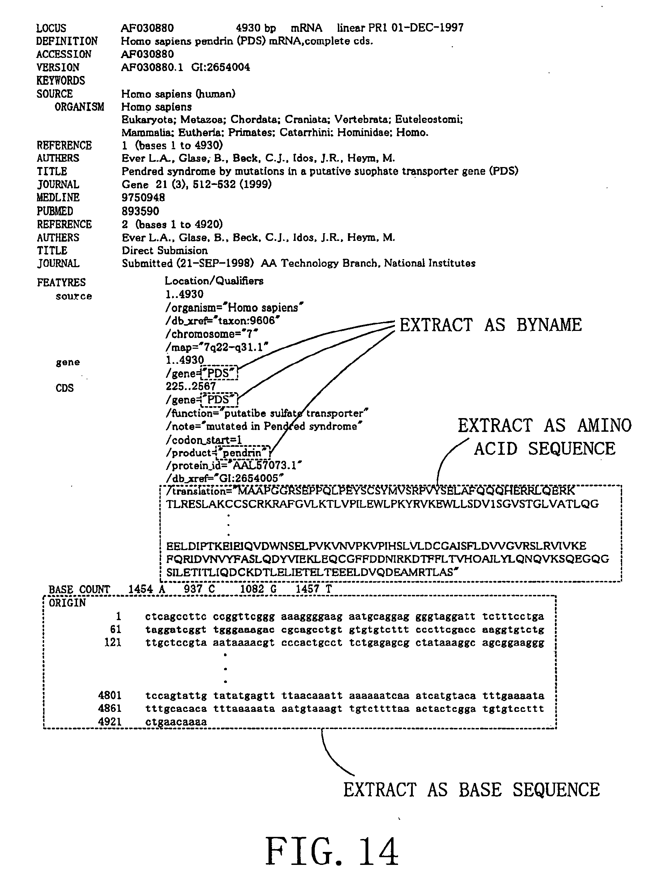

In the general search using Web browser, when information relating to the GI number "2654004" is searched from Entrez, the result as illustrated in FIG 14 can be obtained. As illustrated in FIG 14, the obtained result includes a gene name, a protein name, a species, document information, information of a base sequence, an amino acid sequence. Moreover, in the gene and product fields, information relating to a gene or protein name is described. In this case, those except "pendrin" can be regarded as bynames. Furthermore, a base sequence is shown in an ORIGIN filed, and an amino acid sequence is shown in a translation field.

The condition setting section 16 returns the extraction condition 161 in response to the supplied condition request 141. At the second searching time, it is designated by the extraction condition 161 that the bynames described in the gene and product fields are extracted, the base sequence described in the ORIGIN field is extracted and the amino acid sequence described in the translation field is extracted.

The analyzing section 14 extracts the byname "PDS" described in the gene field, the base sequence described in the ORIGIN field and the amino acid sequence described in the translation field, respectively from the result obtained form Entrez according to the supplied extraction condition 161. Since sequence information is long, they are omitted in the explanation below. The analyzing section 14 supplies the analyzing result 142 including the extracted information to the output control section 15.

When receiving the analyzing result 142 from the analyzing section 14, the output control section 15 determines whether the flag set is already provided. At the second searching time, the flag set is provided. The output control section 15 holds the analyzing result 142. The output control section 15 detects biological information corresponding to the output flag showing "non-obtained" from the held analyzing result 142. The output control section 15 changes the output flag, which corresponds to the detected biological information, to a state showing "obtained." In this case, the analyzing result 142 includes the gene or protein byname obtained from Entrez. For this reason, the output control section 15 changes the output flag 1 from "non-obtained" to "obtained."

The output control section 15 determines whether all output flags show "obtained." At this point, the output flag 3 shows "non-obtained." For this reason, the output control section 15 extracts the name "PSD" and the sequences "base sequence and amino acid sequence" from the held analyzing result 142. The output control section 15 brings the extracted biological information into correspondence with the kind and supplies the result as a re-search query 151 to the query generating section 12.

The query generating section 12 generates a DB query 121 in response to the re-search query 151 from the output control section 15, similar to the above.

At a third searching time, among the search flags corresponding to the combination included in the DB query 121, Entrez 1 flag, Entrez 2 flag and LocusLink1 flag show "searched" and BLAST1 flag shows "unsearched."

The DB query 121 rewrites the contents of the combination, which corresponds to the search flag of "searched", to information indicating "searched", thereby generating the DB query 121 showing the BLAST location, the base sequence and the amino acid sequence.

The query generating section 12 determines whether all corresponding search flags show "searched." As mentioned above, BLAST1 flag shows "non-searched." For this reason, the query generating section 12 changes BLAST1 flag from "non-searched" to "searched." After that, the query generating section 12 supplies the generated DB query 121 to the biological information searching section 13.

The biological information searching section 13 transmits the query signal 131 including the keywords (base sequence and amino acid sequence) to the BLAST location shown by the DB query 121. As a result, the third search is executed using BLAST. At the third searching time, the gene and protein names relating to the base sequence and amino acid sequence as the keywords can be obtained.

The biological information searching section 13 holds the searching result 132 supplied from the DB server. The biological information searching section 13 generates a DB searching result 133 by using the searching result 132, similar to the above. The biological information searching section 13 supplies the generated DB searching result 133 to the analyzing section 14.

The analyzing section 14 supplies the condition request 141 to the condition setting section 16 in response to the DB searching result 133 from the biological information searching section 13. At the third searching time, the analyzing section 14 supplies the condition request 141, which shows that no error occurs, BLAST is used, the kind of keyword is the base sequence or amino acid sequence.

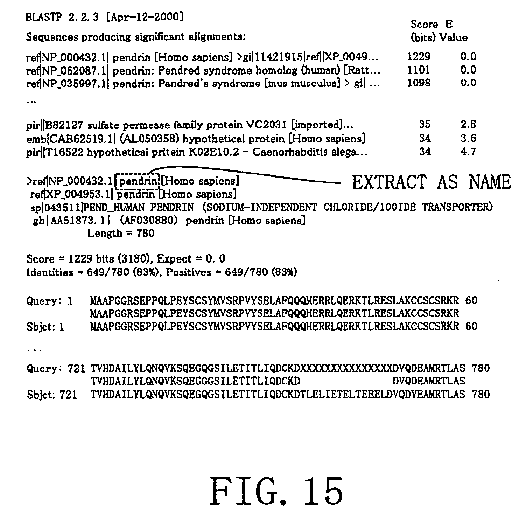

In the general search using Web browser, when homology is searched using BLAST, the result as illustrated in FIG 15 can be obtained. As illustrated in FIG 15, in the obtained result, the base sequences or amino acid sequences, which are homologous with the base or amino acid sequences as the keyword, are listed. Furthermore, in connection with each of the listed sequences, information, which includes a name, an accession number, e-value showing degree of homology and a portion having high homology, is shown.

The condition setting section 16 returns the extraction condition 161 in response to the supplied condition request 141. At the third searching time, it is designated by the extraction condition 161 that the name of the gene or protein, whose sequence has a high homology, is extracted from the result obtained from BLAST.

The analyzing section 14 extracts the name of the gene or protein, whose sequence has a high homology, from the result obtained from BLAST according to the supplied extraction condition 161. The analyzing section 14 supplies the analyzing result 142 including the extracted information to the output control section 15.

When receiving the analyzing result 142 from the analyzing section 14, the output control section 15 determines whether the flag set is already provided. At the third searching time, the flag set is provided. The output control section 15 holds the analyzing result 142. The output control section 15 detects biological information corresponding to the output flag showing "non-obtained" from the held analyzing result 142. The output control section 15 changes the output flag, which corresponds to the detected biological information, to a state showing "obtained." In this case, the analyzing result 142 includes the gene or protein name obtained from BLAST. For this reason, the output control section 15 changes the output flag 3 from "non-obtained" to "obtained."

The output control section 15 determines whether all output flags show "obtained." At this point, all output flags show "obtained." As a result, the output control section 15 outputs all held analyzing results 142 as output information 152.

The above showed the case in which the output control section 15 output all held analyzing results 142 as output information 152 at one time when all output flags showed "obtained." However, the output control section 15 may output output information 152 every time when receiving the analyzing result 142. Moreover, the output control section 15 may add information showing the completion of search to output information 152 when all output flags shows "obtained."

The following will explain a biological information search system and biological information search method according to a second embodiment of the present invention with reference to the drawings.

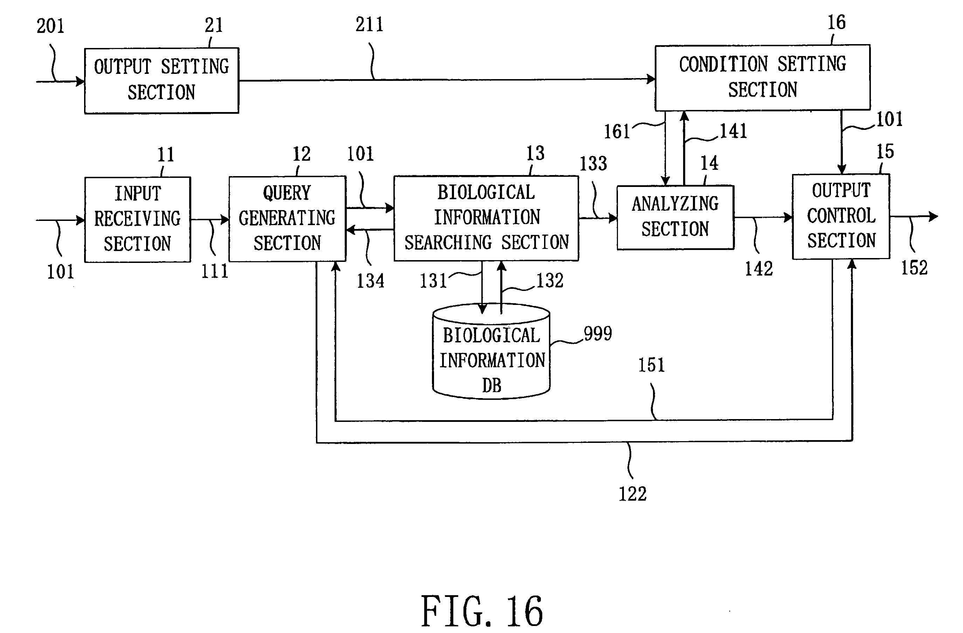

The biological information search system of the second embodiment includes an output setting section 21 in addition to the structural components explained in the first embodiment, as illustrated in FIG 16. Accordingly, the following will mainly explain the output setting section 21.

The output setting section 21 is provided in order that the user can designate information to be extracted from the DB searching result 133 and the condition for outputting the analyzing result 142. This makes it possible for the user to designate which biological information D999 is used to execute a search and what kind of information is extracted or output.

The output setting section 21 receives contents (user designation) 201 that are designated by the user using the input device. The user designation 201 includes information indicating biological information DB 999 (target DB) to be used and information indicating the kind of biological information (kind of target information) to be extracted or output.

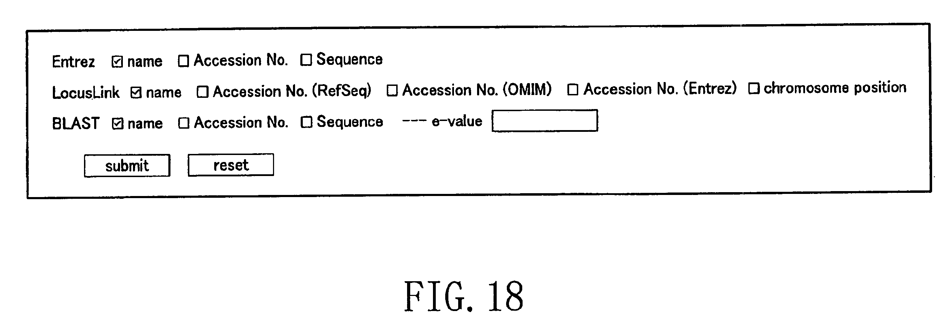

The output setting section 21 includes data of an input screen where multiple condition designation fields for designating the target DB and the kind of target information as illustrated in, for example, FIG 18. The output setting section 21 controls the display to display the input screen, and the user designates the target DB and the kind of target information on the input screen displayed by the input device. As a result, the user designation 201 is supplied to the output setting section 21.

The output setting section 21 decides the target DB and the kind of target information based on the user designation 201, and brings the target DB into correspondence with information indicating the kind of target information. At this time, when the number of target DB and that of kinds of target information are multiple, a plurality of combinations {target DB, kind of target information} are generated.

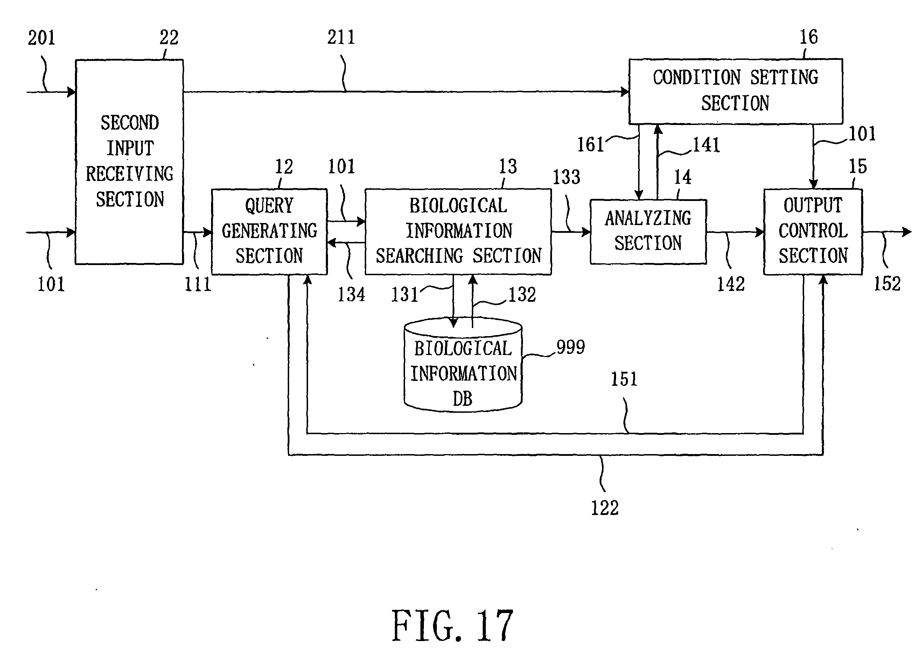

The output setting section 21 supplies a list of combinations {target DB, kind of target information} to the condition setting section 16. In addition, both the output setting section 21 and the input receiving section 11 are user interface. Accordingly, as illustrated in FIG 17, a second input receiving section 22 having both functions may be provided in place of the output setting section 21 and the input receiving section 11.

An explanation will be next given of a specific example of operations the biological information search system according to the second embodiment.

In this example, the user designates three points, namely, obtaining a gene or protein byname from Entrez; obtaining a gene or protein byname from LocusLink; and obtaining byname of a gene or protein having homology from BLAST.

Additionally, the configuration components except the output setting section 21 are the same as those of the first embodiment. Accordingly, the following will mainly explain the output setting section 21.

The output setting section 21 controls the display and the like and displays an input screen as illustrated in, for example, FIG 18.

The user marks a checkbox corresponding to each of "name" of Entrez, "name" of LocusLink, and "name" of BLAST and clicks a submit button. As a result, the output setting section 21 receives the user designation 201 showing the designated contents of the user.

The output setting section 21 decides the target DB and the kind of target information based on the user designation 201, and brings the target DB into correspondence with information indicating the kind of target information. This generates combinations {Entrez, gene or protein byname}, {LocusLink, gene or protein byname} and {BLAST, gene having homology or protein name}.

The output setting section 21 supplies the list of these combinations as output designation information 211 to the condition setting section 16.

In a case where the user inputs a numerical value in an e-value field provided on the input screen, it is possible to extract or output the name of the gene or protein having such homology that is designated by the e-value from the result obtained from BLAST.

The condition setting section 16 supplies the extraction condition 161 and the output condition 162 according to the destination information 211 from the output setting section 21.

The following will explain a biological information search system and biological information search method according to a third embodiment of the present invention with reference to the drawings.

According to the biological information search system of the third embodiment, an actual search is executed after procedure for search is decided.

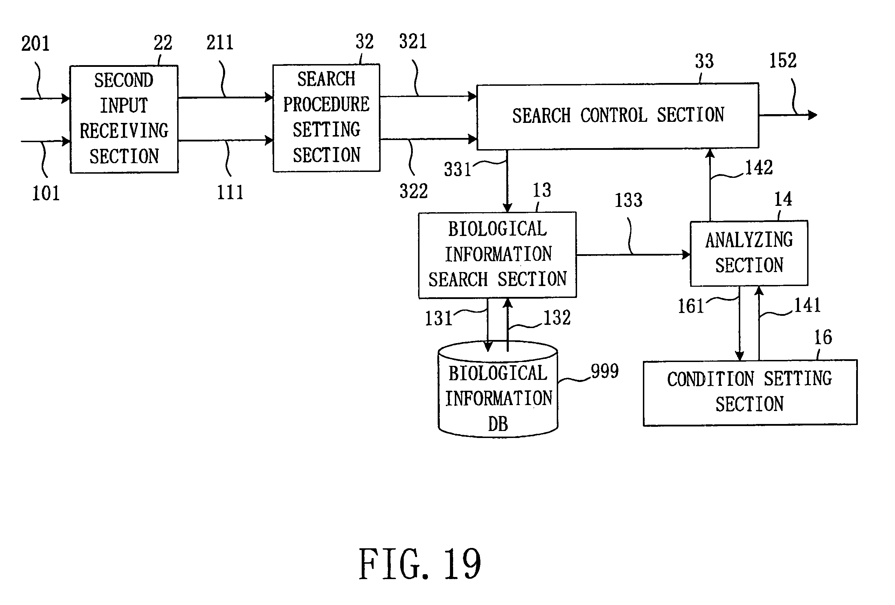

As illustrated in FIG 19, the biological information search system according to the third embodiment includes a second input receiving section 22, a search procedure setting section 32, a search control section 33, a biological information searching section 13, an analyzing section 14, and a condition setting section 16. In addition, as illustrated in FIG 19, the second input receiving section 22, the biological information searching section 13, the analyzing section 14 and the condition setting section 16 are the same as those of the first or second embodiment. Accordingly, the following will mainly explain the operations of the search procedure setting section 32 and the search control section 33.

The user query 111 and the designation information 211 are supplied to the search procedure setting section 32 from the second input receiving section 22. The search procedure setting section 32 has biological DB information. The biological DB information indicates a location (DB location) of each biological information DB, a kind of keyword that can be used in each biological information DB 999 and a kind of biological information that can be searched from each biological information DB 999. Namely, the biological DB information indicates the list of combinations {DB location, kind of usable keyword, kind of searchable biological information}.

The search procedure setting section 32 compares each kind of keyword shown by the user query 111 with each kind of usable keyword shown by the biological DB information. The search procedure setting section 32 obtains a combination, which shows the same kind of usable keyword as the kind of keyword shown by each combination of the user query 111, from the biological DB information. The search procedure setting section 32 generates a DB query list 321 by using the obtained combination. The DB query list 321 shows the list of combinations {DB location, kind of usable keyword, kind of searchable biological information}. Each combination of the DB query list 321 corresponds to biological information DB 999 used at time of the first searching. The search procedure setting section 32 holds the generated DB query list 321.

The search procedure setting section 32 determines whether the generated DB query list 321 satisfies the condition shown by the designation information 211. More specifically, the search procedure setting section 32 detects the combination, which shows the location of the target DB indicated by each combination of the designation information 211 and the kind of searchable biological information, which is the same as the kind of target information indicated by each combination of designation information 211, from the DB query list 321. Namely, the search procedure setting section 32 detects the combination corresponding to each combination of the designation information 211 from the DB query list 321. In a case where all corresponding combinations are detected, the search procedure setting section 32 determines that the generated DB query list 321 satisfies the condition. On the other hand, in a case where at least one corresponding combination is not detected, the search procedure setting section 32 does not determine that the generated DB query list 321 satisfies the condition.

In a case where search procedure setting section 32 determines that the generated DB query list 321 satisfies the condition shown by the designation information 211, the search procedure setting section 32 supplies the generated DB query list 321 to the search control section 33. Moreover, the search procedure setting section 32 sends the supplied user query 111 as user query information 322 to the search control section 33.

In a case where search procedure setting section 32 does not determine that the generated DB query list 321 satisfies the condition shown by the designation information 211, the search procedure setting section 32 obtains a predetermined combination from the DB query list 321. The search procedure setting section 32 extracts information, which indicates the kind of searchable biological information, from the obtained combination. The search procedure setting section 32 obtains a combination, which shows the same kind of usable keyword as the kind indicated by the extracted information, from the biological DB information. The search procedure setting section 32 adds the obtained combination to the DB query list 321. The added combination corresponds to the biological information DB 999 used at the re-searching time.

After adding the new combination to the DB query list 321, the search procedure setting section 32 determines whether the generated DB query list 321 satisfies the condition shown by the designation information 211, similar to the above. Then, the search procedure setting section 32 adds a new combination to the DB query list 321 until the condition is satisfied. As a result, a series of searching procedure is decided.

The search procedure setting section 32 supplies the generated DB query list 321 to the search control section 33. At this time, the supplied user query 111 as user query information 322 is supplied to the search procedure setting section 32.

In the above explanation, the DB query list 321 was generated from the user query 111 and the biological DB information, and the new combination was added to the DB query list 321. However, the DB query list 321 is generated from the designation information 211 and the biological DB information, so that the new combination may be added to the DB query list 321.

In this case, the search procedure setting section 32 compares each kind of target information shown by designation information 211 with each kind of searchable biological information shown by the biological DB information. The search procedure setting section 32 obtains a combination, which shows the same kind of biological information as the kind of object information shown by each combination of designation information 211, from the biological DB information. The search procedure setting section 32 generates a DB query list 321 by using the obtained combination. Each combination of the DB query list 321 corresponds to biological information DB 999 used at the last searching time.

Then, the search procedure setting section 32 determines that the generated DB query list 321 satisfies the condition shown by the designation information 211. More specifically, the search procedure setting section 32 detects the combination, which shows the same kind of usable keyword as the kind of keyword shown by each combination of the user query 111, from the DB query list 321. Namely, the search procedure setting section 32 detects the combination corresponding to each combination of the user query 111 from the DB query list 321. In a case where all corresponding combinations are detected, the search procedure setting section 32 determines that the DB query list 321 satisfies the user query 111. On the other hand, in a case where at least one corresponding combination is not detected, the search procedure setting section 32 does not determine that the DB query list 321 satisfies the user query 111.

In a case where search procedure setting section 32 determines that the DB query list 321 satisfies the user query 111, the search procedure setting section 32 supplies the generated DB query list 321 to the search control section 33. Moreover, the search procedure setting section 32 sends the supplied user query 111 as user query information 322 to the search control section 33.

In a case where search procedure setting section 32 does not determine that the generated DB query list 321 satisfies the user query 111, the search procedure setting section 32 obtains a predetermined combination from the DB query list 321. The search procedure setting section 32 extracts information, which indicates the kind of usable keyword, from the obtained combination. The search procedure setting section 32 obtains a combination, which shows the same kind of searchable biological information as the kind indicated by the extracted information, from the biological DB information. The search procedure setting section 32 adds the obtained combination to the DB query list 321. The added combination corresponds to the biological information DB 999 used at the time of one previous search.

After adding the new combination to the DB query list 321, the search procedure setting section 32 determines whether the generated DB query list 321 satisfies the user query 111, similar to the above. Then, the search procedure setting section 32 repeats addition processing until the DB query list 321 satisfies the user query 111.

Moreover, two DB query lists 321 are generated using both the designation information 211 and the user query 111, so that combinations may be added in such a way that two DB query lists 321 conform to each other.

The DB query list 321 and user query information 322 are supplied to the search control section 33 from the search procedure setting section 32. The search control section 33 obtains a combination corresponding to the first search from the supplied DB query list 321. The search control section 33 generates a DB query 331 for the first search by use of the obtained combination and the user query information 322. The DB query 331 shows the list of combinations {DB location, keyword, kind of keyword}. In addition, the DB location is expressed by, for example, URL, similar to the first and second embodiments. The search control section 33 supplies the generated DB query 331 to the biological information searching section 13.

The configuration and operation of the biological information searching section 13 and the analyzing section 14 are substantially the same as those of the first and second embodiments. Moreover, the configuration and operation of the condition setting section 16 are substantially the same as those of the first and second embodiments. The analyzing section 14 generates analyzing result 142 by using the DB searching result 133 obtained from each biological information DB 999 at the first searching time. Then, the analyzing section 14 supplies the generated analyzing result 142 to the search control section 33.

The search control section 33 holds the analyzing result 142 supplied from the analyzing section 14. The search control section 33 determines whether all searches corresponding to the combinations included in the DB query list 321 are conducted.

In a case where the search control section 33 determines that all searches are conducted, the search control section 33 outputs all analyzing results 142 supplied from the analyzing section 14 as output information 152.

In a case where the search control section 33 does not determine that all searches are conducted, the search control section 33 obtains a combination corresponding to a next search from the DB query list 321. The search control section 33 extracts biological information of the kind of usable keyword shown by each obtained combination from the held analyzing result 142. The search control 33 generates a DB query 331 for re-search by use of the obtained combination and the extracted biological information. The search control 33 supplies the generated DB query 331 to the biological information searching section 13.

Afterward, the aforementioned processing is repeated until all searches corresponding to the combinations included in the DB query list 321 are conducted.

Next, the following will specifically explain processing performed by the search procedure setting section 32.

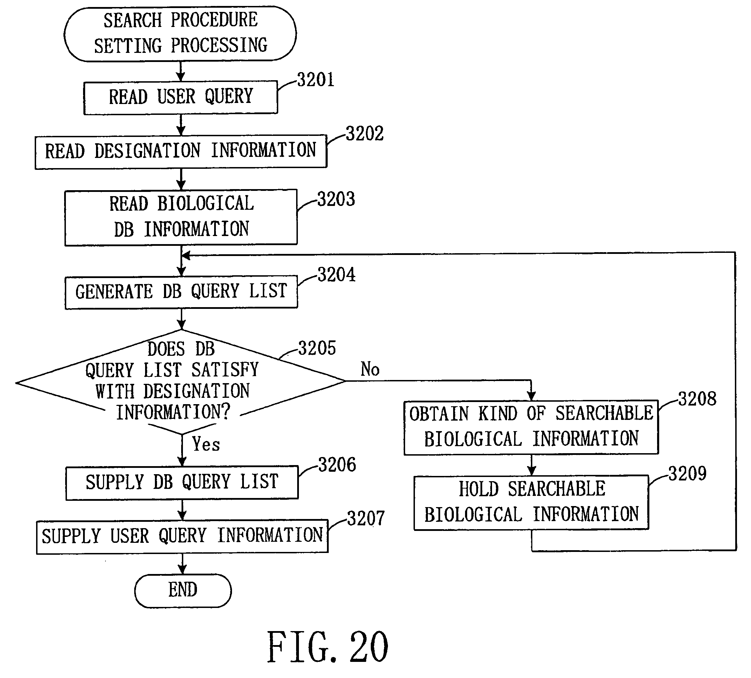

FIG 20 is a flowchart illustrating processing performed by the search procedure setting section 32.

The search procedure setting section 32 reads the user query 111 {keyword, kind of keyword} supplied from the second input receiving section 22 (step 3201).

Sequentially, the search procedure setting section 32 reads the designation information 211 {target DB, kind of target information} supplied form the second input receiving section 22 (step 3202).

The search procedure setting section 32 reads the held biological DB information {DB location, kind of usable keyword, kind of searchable biological information} (step 3203).

The search procedure setting section 32 obtains a combination, which shows the same kind of usable keyword as the kind of keyword shown by each combination of the user query 111, from the biological DB information. The search procedure setting section 32 generates a DB query list 321 by using the obtained combination (step 3204). The DB query list 321 shows the list of combinations {DB location, kind of usable keyword, kind of searchable biological information}.

The search procedure setting section 32 determines whether the generated DB query list 321 satisfies the condition shown by the designation information 211 (step 3205). More specifically, as mentioned above, the search procedure setting section 32 determines whether all combinations corresponding to the respective combinations of the designation information 211 are detected from the DB query list 321.

In a case where the search procedure setting section 32 determines that the DB query list 321 satisfies the condition shown by the designation information 211 (step 3205; Yes), the search procedure setting section 32 supplies the generated DB query list 321 to the search control section 33 (step 3206).

Sequentially, the search procedure setting section 32 supplies the supplied user query 111 as user query information 322 to the search control section 33 (step 3207).

On the other hand, in a case where the search procedure setting section 32 does not determine that the DB query list 321 satisfies the condition shown by the designation information 211 (step 3205; No), the search procedure setting section 32 obtains a combination corresponding to the last search from the DB query list 321. The search procedure setting section 32 extracts information, which indicates the kind of searchable biological information, from the obtained combination (step 3208).

The search procedure setting section 32 holds the extracted information indicating the kind of searchable biological information (step 3209) and the processing returns to step 3204.

In step 3204, the search procedure setting section 32 detects a combination, which shows the same kind of usable keyword as the kind indicated by the extracted information, from the biological DB information. The search procedure setting section 32 obtains a combination that is not included in the DB query list 321 from the detected combinations. The search procedure setting section 32 adds the obtained combination to the DB query list 321. This generates a new DB query list 321. The added combination corresponds to biological information DB 999 used at the re-searching time.

The above processing is repeated until the DB query list 321 satisfies the condition shown by the designation information 211. In addition, the number of times for which the new combination is added to the DB query list 321 may be preset. Or, this may be designated by the user.

Additionally, in step 3205, when there is a plurality of combinations showing the same kind of searchable biological information, the search procedure setting section 32 may use one of them typically.

Next, the following will specifically explain processing performed by the search control section 33.

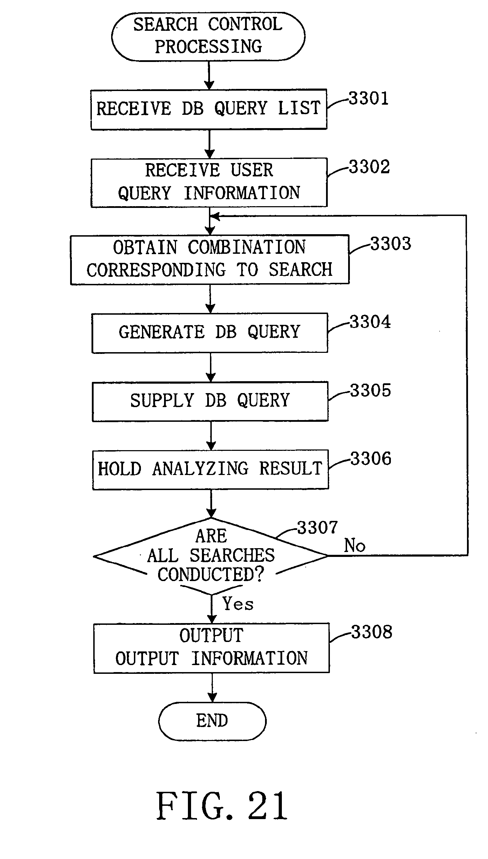

FIG 21 is a flowchart illustrating processing performed by the search control section 33.

The search control section 33 receives a DB query list 321 supplied from the search procedure setting section 32 (step 3301).

After that, the search control section 33 receives a DB query list 322 supplied from the search procedure setting section 32 (step 3302).

The search control section 33 obtains a combination corresponding to the first search from the supplied DB query list 321 (step 3303).

The search control section 33 generates a DB query 331 for the first search by use of the obtained combination and the user query information 322 (step 3304). The DB query 331 shows the list of combinations {DB location, keyword, kind of keyword}.

The search control section 33 supplies the generated DB query 331 to the biological information searching section 13 (step 3305). As a result, transmission of the query signal 131 by the biological information searching section 13 and analysis on the DB searching result 133 by the analyzing section 14 are performed, similar to the first and second embodiments. The analyzing section 14 supplies the analyzing result 142 to the search control section 33.

The search control section 33 holds the analyzing result 142 supplied from the analyzing section 14 (step 3306).

The search control section 33 determines whether all searches corresponding to the combinations included in the DB query list 321 are conducted (step 3307).

In a case where the search control section 33 determines that all searches are conducted (step 3307; Yes), the search control section 33 outputs all held analyzing results 142 as output information 152 (step 3308).

On the other hand, in a case where the search control section 33 does not determine that all searches are conducted (step 3307; No), the search control section 33 returns to step 3303. In step 3303, the search control section 33 obtains a combination corresponding to a next search from the DB query list 321. Then, in step 3304, the search control section 33 extracts biological information of the kind of usable keyword shown by each obtained combination from the held analyzing result 142. The search control section 33 generates a DB query 331 for re-search by use of the obtained combination and the extracted biological information. Afterward, the same processing as mentioned above is performed. The above processing is repeated until it is determined that all searches corresponding to combinations included in the DB query list 321 are performed.

The following will explain the operations of biological information system according to the third embodiment by using the specific example.

In this specific example, similar to the first embodiment, the user inputs a gene or protein name "pendrin." Moreover, similar to the second embodiment, the user marks a checkbox corresponding to each of "name" of Entrez, "name" of LocusLink, and "name" of BLAST and clicks a submit button on the input screen shown in FIG 18.

Additionally, in the third embodiment, the second input receiving section 22, the biological information searching section 13, the analyzing section 14 and the condition setting section 16 are the same as those of the first and second embodiments. For this reason, the following will mainly explain the operations of the search procedure setting section 32 and the search control section 33.

The search procedure setting section 32 reads the user query 111 and the designation information 211 which are supplied from the second input receiving section 22. In this case, the user query 111 shows {keyword, kind of keyword} = {pendrin, gene or protein name}. The designation information 211 indicates {target DB, kind of target information} = {Entrez, gene or protein name (byname)}, {LocusLink, gene or protein name (byname)}, { BLAST, name (byname) of gene or protein having homology }.

Sequentially, the search procedure setting section 32 reads the held biological DB information. In this example, the biological DB information shows the following combinations {DB location, kind of usable keyword, kind of searchable biological information}.
Entrez 11= {Entrez location 1, gene or protein name, gene or protein byname}
Entrez 12= {Entrez location 2, gene or protein name, GI number}
Entrez 13 = {Entrez location 3, GI number, base or amino acid sequence}
LocusLink 11 = {LocusLink location 1, gene or protein name, gene or protein byname}
BLAST 11 = {BLAST location 1, sequence information, name of gene or protein having homology}

The search procedure setting section 32 obtains a combination, which shows the same kind of usable keyword as the kind of keyword shown by each combination of the user query 111, from the biological DB information. In this case, Entrez 11, Entrez 12, and LocusLink 11 are obtained from the biological DB information. The search procedure setting section 32 generates a DB query list 321 by using the obtained combinations.

The generated DB query list 321 {location, usable kind, searchable kind} shows the following combinations.
Query (1, 1)= {Entrez location 1, gene or protein name, gene or protein byname}
Query (1, 2) = {Entrez location 2, gene or protein name, GI number}
Query (1, 3) = {LocusLink location 1, gene or protein name, gene or protein byname}

The search procedure setting section 32 determines whether the generated DB query list 321 satisfies the condition shown by the designation information 211. More specifically, the search procedure setting section 32 determines whether all combinations corresponding to the respective combinations of the designation information 211 are detected from the DB query list 321. In this case, the combination corresponding to the combination of designation information 211 {BLAST, name of gene or protein having homology} is not included in the DB query list 321. For this reason, the search procedure setting section 32 does not determine that the generated DB query list 321 satisfies the condition shown by the designation information 211.

Then, the search procedure setting section 32 obtains a combination corresponding to the last search from the DB query list 321. At this point, all combinations of the DB query list 321 correspond to the first search. Accordingly, the search procedure setting section 32 obtains query (1, 1), query (1, 2), and query (1, 3). The search procedure setting section 32 extracts information indicating the kind of searchable biological information from the obtained combination. In this case, "gene or protein name" and "GI number" are extracted.

The search procedure setting section 32 holds the extracted information. The search procedure setting section 32 detects a combination, which shows the same kind of usable keyword as the kind indicated by the extracted information, from the biological DB information. The search procedure setting section 32 obtains a combination that is not included in the DB query list 321 from the detected combinations. In this case, Entrez 13 is obtained from the biological DB information.

The search procedure setting section 32 adds the obtained combination to the DB query list 321 as query (2, 1) = {Entrez location 3, GI number, base or amino acid sequence}. Query (2, 1) corresponds to the second search. As a result, a new DB query list 321 is configured as follows.
{Query (1, 1)}
{Query (1, 2), Query (2, 1)}
{Query (1, 3)}

After that, the search procedure setting section 32 determines whether the new DB query list 321 satisfies the condition shown by the designation information 211, similar to the above. In this case, the combination corresponding to the combination of designation information 211 {BLAST, name of gene or protein having homology} is not included in the DB query list 321, either. For this reason, the search procedure setting section 32 does not determine that the generated DB query list 321 satisfies the condition shown by the designation information 211.

The search procedure setting section 32 obtains a combination corresponding to the last search from the DB query list 321, similar to the above. In this case, the search procedure setting section 32 obtains query (2, 1) corresponding to the second search from the DB query list 321. The search procedure setting section 32 extracts information, which shows the kind of searchable biological information, from the obtained combination. In this case, "base or amino acid sequence" is obtained.

The search procedure setting section 32 holds the extracted information. The search procedure setting section 32 detects a combination, which shows the same kind of usable keyword as the kind shown by the extracted information, from the biological DB information. The search procedure setting section 32 obtains a combination that is not included in the DB query list 321 from the detected combinations. In this case, BLAST 11 is obtained from the biological DB information.

The search procedure setting section 32 adds the obtained combination to the DB query list 321 as query (3, 1) = {BLAST location 1, sequence information, name of gene or protein having homology}. Query (3, 1) corresponds to the third search. As a result, a new DB query list 321 is configured as follows.
{Query (1,1)}
{Query (1, 2), Query (2, 1), Query (3, 1)}
{Query (1, 3)}

After that, the search procedure setting section 32 determines whether the new DB query list 321 satisfies the condition shown by the designation information 211, similar to the above. In this case, all combinations corresponding to the combinations of the designation information 211 are included in the DB query list 321. For this reason, the search procedure setting section 32 determines that the generated DB query list 321 satisfies the condition shown by the designation information 211.

Thereafter, the search procedure setting section 32 supplies the generated DB query list 321 to the search control section 33. Moreover, the search procedure setting section 32 supplies the supplied user query 111 as user query information 322 to the search control section 33.

The search control section 33 receives the DB query list 321 and user query information 332 supplied from the search procedure setting section 32.

The search control section 33 obtains a combination corresponding to the first search from the supplied DB query list 321. In this case, query (1, 2), query (2, 1) query (1, 3) are obtained.

The search control section 33 generates a DB query 331 for the first search by use of the obtained combinations and the user query information 322. More specifically, the search control section 33 extracts information, which indicates the DB location, from each obtained combination, and extracts information, which indicates the keyword and the kind of the keyword, from the user query information 322. The search control section 33 brings the extracted information into correspondence with each other to generate a DB query 331. For example, the combination of DB query 331 {Entrez location 1, pendrin, gene or protein name} is generated from query (1, 1) = {Entrez location 1, gene or protein name, gene or protein byname}. The same can be applied to query (1, 2) and query (1, 3).

The search control section 33 supplies the generated DB query 331 to the biological information searching section 13. As a result, the first search is conducted. The analyzing section 14 supplies the analyzing result 142 to the search control section 33. By the first search, information such as byname "PDS", GI number "2654004" and the like is obtained from Entrez. Moreover, information such as byname "SLC26A4, DFNB4, PDS, pendrin" and the like is obtained from LocusLink.

The search control section 33 holds the analyzing result 142 supplied from the analyzing section 14. The search control section 33 determines whether all searches corresponding to the combinations included in the DB query list 321 are conducted. In this case, searching to query (2, 1) and query (3, 1) is not performed. For this reason, the search control section 33 does not determine that all searches are conducted.

The search control section 33 obtains a combination corresponding to the second search from the DB query list 321. In this case, query (2, 1) is obtained. The search control section 33 extracts the biological information (GI number), which is the kind of usable keyword shown by query (2, 1), from the held analyzing result 142. The search control section 33 generates a DB query 331 for the second search by use of query (2, 1) and the extracted biological information. Then, the search control section 33 supplies the generated DB query 331 to the biological information searching section 13.

As a result, the second search is conducted. The analyzing section 14 supplies the analyzing result 142 to the search control section 33. By the second search, information such as "base or amino acid sequence" and the like is obtained from Entrez.

The search control section 33 holds the analyzing result 142 supplied from the analyzing section 14. The search control section 33 determines whether all searches corresponding to the combinations included in the DB query list 321 are conducted, similar to the above. In this case, searching to query (3, 1) is not performed. For this reason, the search control section 33 does not determine that all searches are conducted.

The search control section 33 obtains a combination corresponding to the third search from the DB query list 321. In this case, query (3, 1) is obtained. The search control section 33 generates a DB query 331 for the third search by use of query (3, 1) and the held analyzing result 142. Then, the search control section 33 supplies the generated DB query 331 to the biological information searching section 13.

As a result, the third search is conducted. The analyzing section 14 supplies the analyzing result 142 to the search control section 33. By the third search, information such as "name of gene or protein having homology" and the like is obtained from BLAST.

The search control section 33 holds the analyzing result 142 supplied from the analyzing section 14. The search control section 33 determines whether all searches corresponding to the combinations included in the DB query list 321 are conducted, similar to the above. In this case, the search procedure setting section 32 determines that all searches are conducted.

The search procedure setting section 32 outputs all held analyzing results 142 as output information 152.

The following will explain a biological information search system and biological information search method according to a fourth embodiment of the present invention with reference to the drawings.

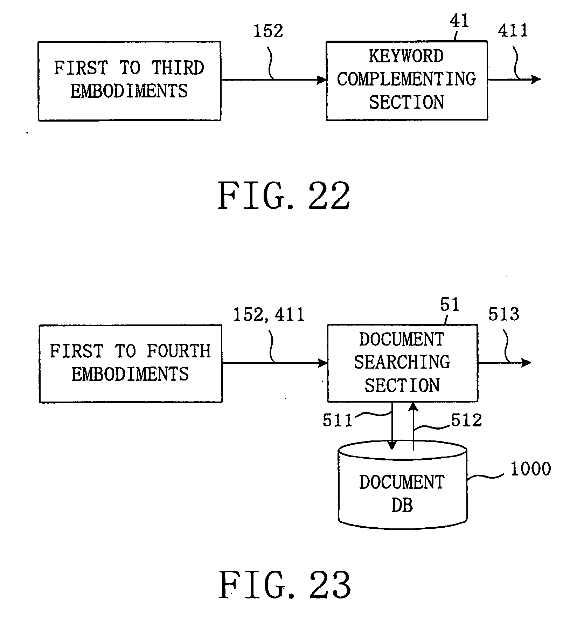

The biological information search system according to the fourth embodiment includes a keyword complementing section 41 in addition to the configuration components illustrated in any of the first to third embodiments, as illustrated in FIG 22.

The output information 152 illustrated in the first to third embodiments is supplied to the keyword complementing section 41. The keyword complementing section 41 complements a gene or protein name to the supplied output information 152. The keyword complementing section 41 outputs the output information 152 having the name complemented as second output information 411.

The configuration components except the keyword complementing section 41 are the same as those of any of the first to third embodiments. Accordingly, the following will mainly explain the keyword complementing section 41.

The keyword complementing section 41 includes the format data of the gene and protein name and data of a complement rule for complementing the name. The keyword complementing section 41 executes complement processing in response to the supplied output information 152.

More specifically, in a case where the gene or protein name included in the output information 152 is formed by alphabetical characters and numerals, and described according to a certain format, the keyword complementing section 41 generates a name, which complies with another format, according to the complement rule. The keyword complementing section 41 adds information of the generated name to the output information 152 and outputs the result as the second output information 411.

A detailed example will be next given of the operations of the keyword complementing section 41.

The following will explain an example when "CA1" as the gene or protein name is included in the output information 152.

The keyword complementing section 41 includes the format data of the gene or protein name such that a numeral is described after the alphabetical characters are continuously described. Moreover, the keyword complementing section 41 includes the data of the complement rule such that a space is inserted between the alphabetical character and the numeral, a hyphen is inserted between the alphabetical character and the numeral and Arabic numerals are changed to Roman numerals.

The keyword complementing section 41 generates "CA 1", "CA-1", "CA I", "CA-I" and the like from "CA1" included in the output information 152, according to the above format and the complement rule in response to the supplied output information 152. The keyword complementing section 41 adds the generated name to the output information 152. The keyword complementing section 41 outputs the output information 152 to which the generated name added as the second output information 411.

The keyword complementing section 41 may further include another format and complement rule except those described above. As the format, the following can be given. For example, a gene or protein name is expressed in such a manner that the alphabetical characters are continuously described and a space is added thereafter and a numeral is described after the space. As the complement rule, the following can be given. For example, a space included in the gene or protein name is deleted; a space is replaced with a hyphen; and Arabic numerals are changed to Roman numerals.

This makes it possible for the keyword complementing section 41 to generate "CA 1", "CA-1", "CA I", "CA-I" and the like from "CA1" according to the above format and the complement rule when the name "CA 1" is included in the output information 152.

In addition, the aforementioned processing is not provided to the name, which is included in the output information 152, which does not comply with the aforementioned format. For example, the gene or protein name "solute carrier family 26, member 4", "p53" and the like do not comply with the aforementioned format. For this reason, the keyword complementing section 41 does not perform the complement processing to such a name. Accordingly, the format and complement rule are appropriately set, thereby making it possible to directly output a name that should not be complemented.

The following will explain a biological information search system and biological information search method according to a fifth embodiment of the present invention with reference to the drawings.

The biological information search system according to the fifth embodiment includes a document searching section 51 in addition to the configuration components illustrated in any of the first to fourth embodiments as illustrated in FIG. 23. Moreover, the biological information search system is connected to multiple DB servers each having a document DB 1000 via a network such as the Internet. Each document DB 1000 includes document data such as scholarly papers, patent publications, and the like. In addition, FIG 23 illustrates one document DB 1000 as an example.

The output information 152 or second output information 411 illustrated in any of the first to fourth embodiments is supplied to the document searching section 51. The document searching section 51 searches a document relating to information included in the output information 152 or 411 from the multiple documents DB 1000 in response to the supplied output information 152 or 411.

The configuration components except the document searching section 51 are the same as those of any of the first to fourth embodiments. Accordingly, the following will mainly explain the document searching section 51.

The document searching section 51 has document DB information. The document DB information indicates a location of each document DB 1000 and the kind of usable keyword in each document DB 1000. The location of the document DB 1000 is expressed by, for example, URL. Moreover, the document searching section 51 includes data of a second extraction condition for extracting predetermined information from a searching result 512 obtained from each document DB 1000. The second extraction condition shows a format of the searching result 512, a part where extracting information is described, a rule for extracting information, and a format of the searching result 512 when an error occurs in the DB server.

The document searching section 51 extracts one keyword for searching a document from the output information 152 or 411 by using the document DB information in response to the output information 152 or 411. The keyword for searching the document includes, for example, a gene name, a protein name, or bynames of these names. Sequentially, the document searching section 51 obtains the location of the document DB 1000, which can use the extracted keywords, from the document DB information.

The document searching section 51 generates a query signal 511, which shows a document search request, using the extracted keyword and the obtained location. The query signal 511 includes the extracted keyword. The document searching section 51 transmits the generated query signal 511 to the obtained location of each obtained document DB 1000.

Each DB server that received the query signal 511 searches the document relating to the keyword shown by the query signal 511. Each DB server transmits the searching result 512 to the document searching section 51.

The document searching section 51 holds the searching result 512 supplied form each DB server. The document searching section 51 extracts information from the held searching result 512, according to the second extraction condition. More specifically, the document searching section 51 extracts information such as a journal name, an author name or an inventor name, an issue date, a main body, an ID number used in the document DB 1000, etc. The document searching section 51 holds the extracted information as a document searching result 513.

The document searching section 51 determines the format and the like of the searching result 152 by using, for example, the second extraction condition. The document searching section 51 determines whether the searching result 152 includes link information for searching detailed information of the document. When determining that the searching result 152 includes link information, the document searching section 51 performs the same re-search processing as mentioned above using the link information as the keyword. When determining that the searching result 152 includes the link information, the document searching section 51 determines whether all usable keywords are extracted from the output information 152 or 411. In a case where the document searching section 51 does not determine that all usable keywords are extracted, the document searching section 51 extracts one keyword from the output information 152 or 411 and performs search processing, similar to the above. When determining that all usable keywords are extracted, the document searching section 51 outputs all held document results 513.

A detailed explanation will be next given of processing performed by the document searching section 51.

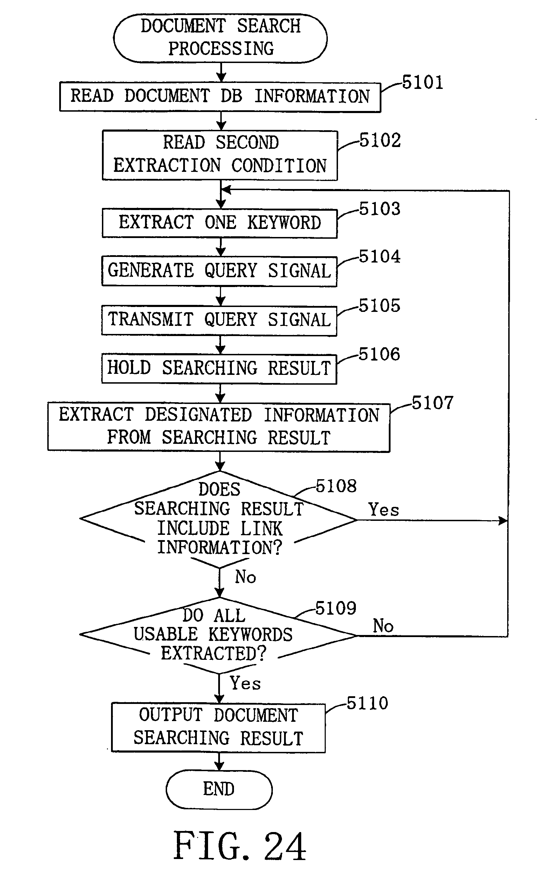

The document searching section 51 starts processing illustrated in FIG 24 in response to the output information 152 or 411.

The document searching section 51 reads held document DB information (step 5101).

Sequentially, the document searching section 51 reads the held second extraction condition (step 5102).

The document searching section 51 extracts one keyword for searching the document from the output information 152 or 411 by using the document DB information (step 5103). More specifically, the document searching section 51 extracts one keyword, which conforms to the kind of a usable keyword shown by the document DB information, from the output information 152 or 411.

The document searching section 51 obtains the location of the document DB 1000, which can use the extracted keyword, from the document DB information. This decides the document DB1000 that is used to search the document. The document searching section 51 generates a query signal 511 by using the extracted keyword and the obtained location (step 5104).

The document searching section 51 transmits the generated query signal 511 to each obtained location (step 5105). As a result, in each DB server that received the query signal 511, the document searching is conducted. The searching result 152 is supplied to the document searching section 51 from each DB server.

The document searching section 51 holds the searching result 152 supplied from each DB server (step 5106).

The document searching section 51 extracts information designated by the second extraction condition from the held document searching result 512. The document searching section 51 holds the extracted information as a document searching result 513 (step 5107).

After that, the document searching section 51 determines whether the searching result 152 includes link information for searching the detailed information of the document, based on the format shown by the second extraction condition (step 5108).

In a case where the document searching section 51 determines that the searching result 152 includes link information (step 5108; Yes), the document searching section 51 goes back to step 5103. In the step 5103, the document searching section 51 extracts link information as a keyword from the searching result 152. The document searching section 51 performs the same processing as mentioned above using the extracted keyword. As a result, the re-search is conducted.

On the other hand, in a case where the document searching section 51 does not determine that the searching result 152 includes link information (step 5108; No), the document searching section 51 determines whether all usable keywords are extracted from the output information 152 or 411 (step 5109).

In a case where the document searching section 51 does not determine that all usable keywords are extracted (step 5109; No), the processing goes back to step 5103. In step 5103, the document searching section 51 extracts one new keyword from the output information 152 or 411 using the document DB information. The document searching section 51 performs the same processing as mentioned above using the extracted keyword. As a result, the document relating to the new word is searched.

On the other hand, in a case where the document searching section 51 determines that all usable keywords are extracted (step 5109; Yes), the document searching section 51 outputs all held document searching results 513 (step 5110). At this time, the document searching section 51 controls the display and the like, so that the list of information shown by all document searching results 513 may be displayed thereon.

Additionally, in step 5107, the document searching section 51 may sequentially output the document searching results 513 without holding the document searching results 513. In this case, the document searching section 51 ends processing without performing step 5110. Moreover, at the time of ending the processing, the document searching section 51 may output information indicating that the document search is completed. This makes it possible to clearly show the user that the document search is completed. Moreover, the user may designate the document DB 1000 to be used and the kind of information to be extracted from the searching result 512.

Next, the following will explain the operations of the document searching section 51 by using a specific example.

In this specific example, the user inputs a gene or protein name "pendrin." Furthermore, a scholarly paper relating to "pendrin" is searched from the document DB1000 "PubMed."

When the user inputs "pendrin", bynames "PDS", "DFNB4", "SLC26A6" and the like are obtained, similar to the first to fourth embodiments. The output information 152 or 411 includes these names.

The document searching section 51 reads the document DB information and the second extraction condition in response to the output information 152 or 411. In this case, the document DB information indicates the location of PubMed and the kind of keyword usable in PubMed.

The document searching section 51 extracts the keyword "pendrin" from the output information 152 or 411 by using the read document DB information. Sequentially, the document searching section 51 obtains the location of "PubMed" from the document DB information. The document searching section 51 generates a query signal 511 using the keyword "pendrin" and the obtained location. In this case, the query signal 511 is generated by combining a searching character string due to CGI with URL of PubMed. A variable provided in a template of the searching character string is replaced with "pendrin." The document searching section 51 transmits the generated query signal 511 to PubMed. As a result, the document relating to "pendrin" is searched from PubMed.

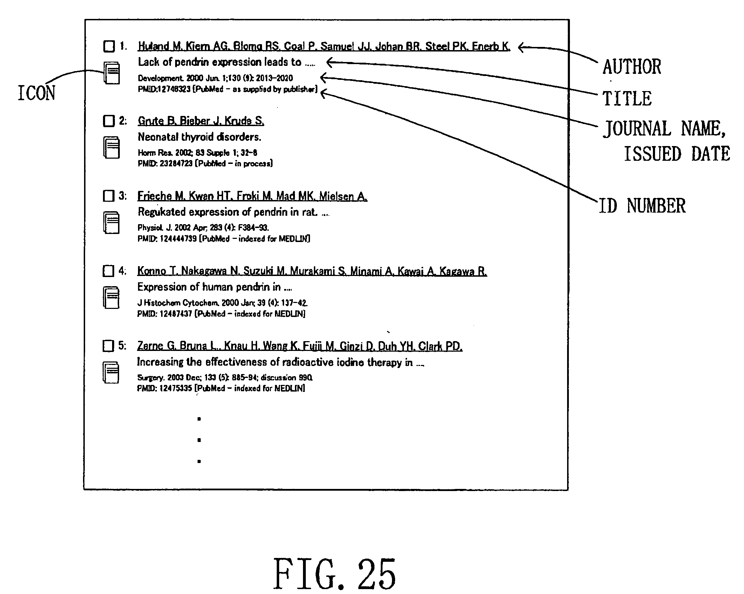

In the general search using Web browser, when the document relating to "pendrin" is searched from PubMed, the result as illustrated in FIG. 25 can be obtained. As illustrated in FIG. 25, in predetermined portions of the obtained result, there are described an author name, a title, a journal name, an issue date, and an ID number used in PubMed. Moreover, regarding the result illustrated in FIG. 25 is configured in such a way that, for example, the detailed information of the document (for example, summary, etc.) can be obtained by clicking the author name or a predetermined icon. Namely, the data of the result shown in FIG 25 includes link information for searching the detailed information of the document.

In this specific example, it is assumed that the searching result 512 from PubMed can be expressed by the same format as that of FIG. 25. Moreover, the searching result 512 is described in HTML. In this case, the information of the document relating to the keyword can be obtained by extracting information described in the predetermined portions of the searching result 512.

The document searching section 51 holds the searching result 511 from PubMed. Sequentially, the document searching section 51 extracts information (author name, title, journal name, etc.) of the document relating to "pendrin" from the predetermined portion of the held searching result 512. The document searching section 51 holds the extracted information as the document searching result 513.

After that, the document searching section 51 determines whether the searching result 152 includes link information for searching the detailed information of the document, based on the format shown by the second extraction condition. As mentioned above, the same searching result 152 as in FIG. 25 includes link information. For this reason, the document searching section 51 determines that the searching result 512 includes link information.

The document searching section 51 extracts the link information (for example, ID in PubMed of each document) as the keyword from the searching result 512. The document searching section 51 performs the same processing as mentioned above, using the extracted keyword. As a result, the re-search is conducted.

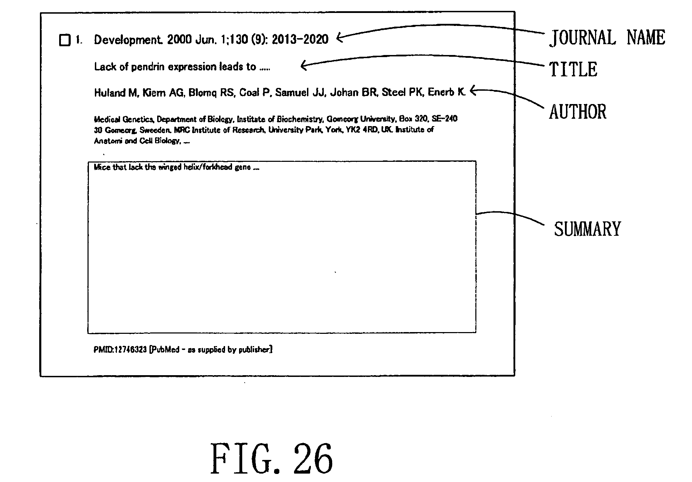

In the general search using Web browser, the result as illustrated in FIG. 26 can be obtained. As illustrated in FIG. 26, in predetermined portions of the obtained result, there are described a summary of the document, an author name, a title, a journal name, and the like. The result of FIG. 26 does not include link information for further obtaining detailed information of the document.

In this detailed example, the searching result 512 obtained by re-searching is expressed by the same format as in FIG. 26. Moreover, the searching result 512 is described in HTML. In this case, the detailed information (summary and the like) of the document can be obtained by extracting information described in the predetermined portions of the searching result 512.

The document searching section 51 holds the searching result 512 from PubMed, similar to the above. Sequentially, the document searching section 51 extracts the detailed information (summary and the like) of the document from the predetermined portions of the held searching result 512. The document searching section 51 holds the extracted information as the document searching result 513.

After that, the document searching section 51 determines whether the searching result 152 includes link information, based on the format shown by the second extraction condition. As mentioned above, the same searching result 152 as in FIG. 26 does not include link information. For this reason, the document searching section 51 does not determine that the searching result 512 includes link information.

Sequentially, the document searching section 51 determines whether all usable keywords are extracted from the output information 152 or 411. In this example, the output information 152 or 411 includes "PDS", "DFNB4", and "SLC26A4" which are bynames of "pendrin"." For this reason, the document searching section 51 does not determine that all usable keywords are extracted. The document searching section 51 extracts one byname as a new keyword from the output information 152 or 411 by using the document DB information. The document searching section 51 performs the same processing as mentioned above using the extracted keyword.

The document searching section 51 performs the same processing to another bynames. This makes it possible to obtain the document relating to biological information included in the output information 152 or 411. When all keywords are extracted from the output information 152 or 411, the document searching section 51 outputs all held document searching result 513.

The following will explain a biological information search system and biological information search method according to a sixth embodiment of the present invention with reference to the drawings.

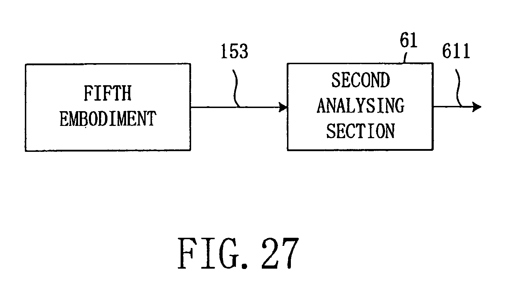

The biological information search system according to the sixth embodiment includes a second analyzing section 61 in addition to the configuration components illustrated in the fifth embodiment as illustrated in FIG.27. The second analyzing section 61 combines or rearranges information included in the document searching result 513 shown by the fifth embodiment. This generates a document analyzing result 611. The second analyzing section 61 outputs the generated document analyzing result 611.

The configuration components except the second analyzing section 61 are the same as those of the fifth embodiment. Accordingly, the following will mainly explain the second analyzing section 61.

The second analyzing section 61 has data showing a rule for arranging information shown by the document searching result 513. As the arrangement rule, the following can be given. For example, overlapping information is combined into one; an index is added to each document; the number of documents is obtained; the documents are sorted in order of publication date, information is classified by journal name; and information is classified by author name.

The document searching result 513 is supplied to the second analyzing section 61 from the document searching section 51. The second analyzing section 61 arranges information included in the document searching result 513, according to the arrangement rule in response to the document searching result 513 from the document searching section 51. More specifically, the second analyzing section 61 rearranges information shown by the document searching result 513, adds an index to each document, and combines overlapping information into one. Moreover, the second analyzing section 61 obtains the number of documents and adds information, which indicates the obtained number of documents, to the document searching result 513. The second analyzing section 61 outputs the arranged document searching result 513 as the document analyzing result 611.

Next, the following will explain the operations of the second analyzing section 61 using a specific example.

In this specific example, the user inputs a gene or protein name "pendrin." By the processing shown in the fourth embodiment, information, which indicates the gene or protein name, is complemented to the output information 152. By the processing shown in the fifth embodiment, scholarly papers relating to "pendrin" and its byname "SLC26A4" are searched from PubMed.

The document searching result 513 relating to the scholarly papers is supplied to the second analyzing section 61 from the document searching section 51.

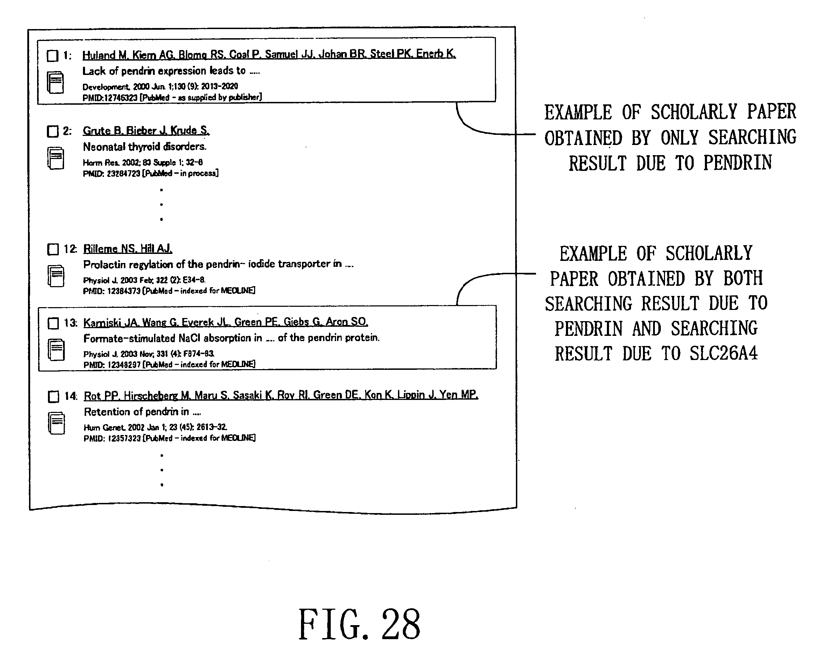

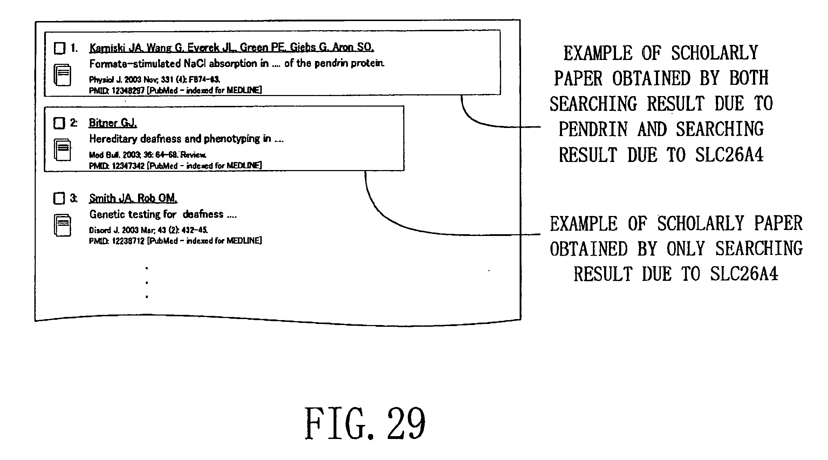

FIG 28 illustrates an example of the document searching result 513 obtained when the document relating to "pendrin" is searched from PubMed. FIG 29 illustrates an example of the document searching result 513 obtained when the document relating to "SLC26A4" complemented by the keyword complementing section 41 is searched from PubMed. As illustrated in FIGS. 28 and 29, the same information is included in two document searching results 513.

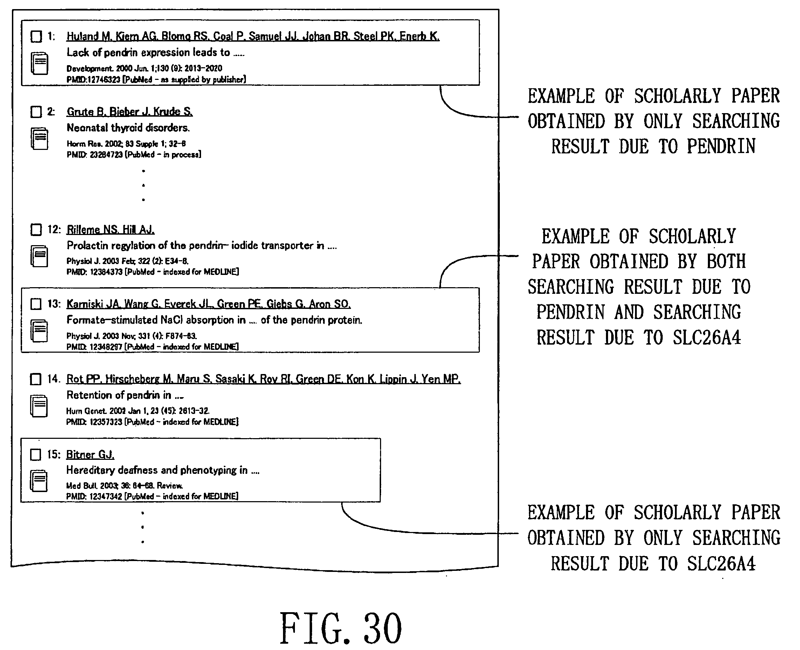

The second analyzing section 61 combines overlapping information included in two document searching results 513 into one, according to the arrangement rule. As a result, the second analyzing section 61 generates a document analyzing result 611 as illustrated in FIG. 30. The second analyzing section 61 outputs the generated document analyzing result 611. At this time, the second analyzing section 61 controls the display and the like, so that the list of information shown by the document analyzing result 611 may be displayed thereon.

The following will explain a biological information search system and biological information search method according to a seventh embodiment of the present invention with reference to the drawings.

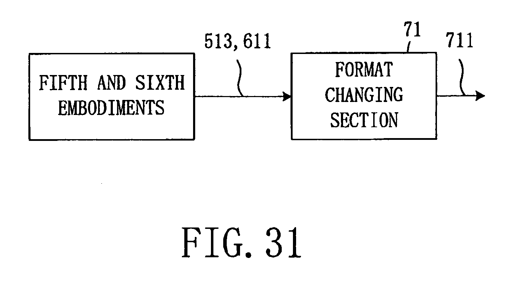

The biological information search system according to the seventh embodiment includes a format changing section 71 in addition to the configuration components illustrated in the fifth or sixth embodiment as illustrated in FIG. 31. The format changing section 71 changes the format of the document searching result 513 shown in the fifth embodiment or the format of the document analyzing result 611 shown in the sixth embodiment. The format changing section 71 outputs a change result 711.

The configuration components except the format changing section 71 are the same as those of the fifth or sixth embodiment. Accordingly, the following will mainly explain the format changing section 71.

The format changing section 71 has data showing a change rule for changing a format of information, which is included in the document searching result 513 or the document analyzing result 611, to a predetermined format. As the change rule, the following can be given. For example, a display color of a keyword designated by a user is changed; a gene or protein name and its byname are displayed together; and a document ID and a summary are displayed in the form of list.

The document searching result 513 is supplied to the format changing section 71 from the document searching section 51. Or, the document analyzing result 611 is supplied thereto from the second analyzing section 61. The format changing section 71 reads the change rule in response to the document searching result 513 or the document analyzing result 611. The format changing section 71 changes the format of information, which is indicated by the document searching result 513 or the document analyzing result 611, according to the read change rule. The format changing section 71 outputs a changing result 711. At this time, the format changing section 71 controls the display and the like, so that the changing result 711 may be displayed thereon.

Next, the following will explain the operations of the format changing section 71 by using a specific example.

In this specific example, the user inputs a gene or protein name "pendrin." By the processing shown in the fourth embodiment, information, which indicates the gene or protein name, is complemented to the output information 152. By the processing shown in the fifth embodiment, a scholarly paper relating to "pendrin" is searched from PubMed.

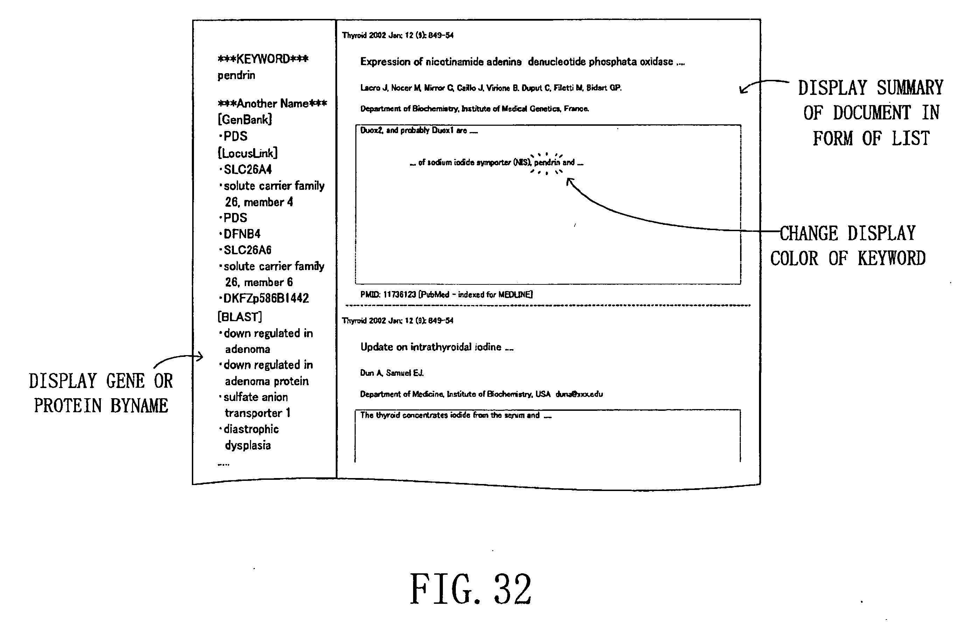

The format changing section 71 reads the change rule in response to the document searching result 513 or the document analyzing result 611. In this case, the change rule shows that "pendrin" and its byname are displayed together and the document ID and the summary are displayed in the form of list. Moreover, the change rule shows that the display color of the keyword "pendrin" is changed. The format changing section 71 changes the format of the information shown by the document searching result 513 or the document analyzing result 611. The format changing section 71 outputs the changing result 711. As a result, the changing result 711 as illustrated in, for example, FIG. 32 is displayed on the display. The document information relating to the keyword is displayed.

As explained in the first to seventh embodiments, the biological information search system that conducts a further search by use of information obtained by the first search. This makes it possible to obtain a searching result with few omissions even if only one gene or protein name is designated. Moreover, the search can be executed regardless of the kind of keyword designated by the researcher. Accordingly, the researchers can designate biological information except the gene or protein name as a keyword. This makes it possible to reduce time, labor, cost that the researchers spend on searching.

Additionally, the above explained the case in which biological information was searched by way of illustration. However, searching information may be information of various fields of science such as chemistry, engineering, literature, history, and the like. In this case, the format, which is generally used in each field, is used. Moreover, the present invention can be applied to any system if the system is one that searches information by using the database. For example, the present invention can be applied to a library search system of the library, an event search system of the police office and the like.

The apparatus of the present invention can be realized by a general computer, without the need for a dedicated apparatus. A program and data for controlling a computer to execute the above-described processes may be recorded on a medium (a floppy disk, CD-ROM, DVD or the like) and distributed, and the program may be installed into the computer and run on an OS (Operating System) to execute the above-described processes, thereby achieving the apparatus of the present invention. The above program and data may be stored in a disk device or the like included in the server device on the Internet, and embedded in a carrier wave. The program and data embedded in the carrier wave may be downloaded into the computer so as to realize the apparatus of the present invention.

Various embodiments and changes may be made thereunto without departing from the broad spirit and scope of the invention. The above-described embodiments are intended to illustrate the present invention, not to limit the scope of the present invention. The scope of the present invention is shown by the attached claims rather than the embodiments. Various modifications made within the meaning of an equivalent of the claims of the invention and within the claims are to be regarded to be in the scope of the present invention.

## Claims

1. An information search system **characterized by** comprising:
an input receiving unit (11) which receives a query to a database from a user, creates a user query for searching information from a database in accordance with the query of the user, and outputs the created user query;
a query generating unit (12) which receives the user query or a re-search query for re-searching information from a database, generates a database query for actually searching information from a database in accordance with the user query or the re-search query, and outputs the created database query;
a searching unit (13) which executes a search of information in accordance with the database query, and outputs an information searching result;
an analyzing unit (14) which analyzes the information searching result output by the searching unit (13) and outputs an information analyzing result;
an output control unit (15) which outputs the information analyzing result from the analyzing unit (14) as output information, creates the re-search query by using the information analyzing result, and supplies the created re-search query to said query generating unit (12); and
a condition setting unit (16) which has an analyzing condition for analyzing the information searching result and an output condition for outputting the output information, supplies the analyzing condition to said analyzing unit (14), and supplies the output condition to said output control unit (15),
**characterized in that** said analyzing unit (14) analyzes the information searching result in accordance with the analyzing condition from said condition setting unit (16); and
said output control unit (15) outputs the output information in accordance with the output condition from said condition setting unit (16).

2. The information search system according to claim 1, **characterized by** further comprising an output setting unit (21) which receives conditions, concerning an analyzing of the information searching result and an output of the output information, from the user, and provides the received conditions to said condition setting unit (16),
**characterized in that** said condition setting unit (16) holds the conditions from said output setting unit (21) as the analyzing condition and the output condition.

3. The information search system according to claim 2, **characterized by** further comprising a document searching unit (51) which receives the output information output by said output control unit (15), searches a document related to the output information from database, and outputs a document searching result.

4. The information search system according to claim 3, **characterized by** further comprising a second analyzing unit (61) which receives the document searching result, analyzes the document searching result by extracting predetermined information from the document searching result, and outputs a document analyzing result.

5. The information search system according to claim 4, **characterized by** further comprising a format changing unit (71) which receives the document analyzing result, changes a format of the document analyzing result into a predetermined format, and outputs a document analyzing result whose format is changed.

6. The information search system according to claim 5, **characterized by** further comprising a complementing unit (41) which receives the output information output by said output control unit (15), and complements the output information with information concerning the output information.

7. The information search system according to claim 6, **characterized in that** the information to be searched is biological information.

8. An information search system **characterized by** comprising:
a second input receiving unit (22) which receives a query to a database and a designation of information to be output, from a user, creates a user query for searching information from a database and a designation information representing the information to be output, in accordance with the query and the designation of the user, and outputs the user query and the designation information;
a search procedure setting unit (32) which sets a search procedure in accordance with the user query and the designation information, and outputs a database query list representing the search procedure;
a search control unit (33) which controls a search of information from the database and an analyzing of an information searching result, in accordance with the database query list and the user query, and outputs an information analyzing result as output information;
a searching unit (13) which executes the search of information under control of said search control unit (33), and outputs the information searching result;
an analyzing unit (14) which executes the analyzing of the information searching result under control of said search control unit (33), and outputs the information analyzing result to said search control unit (33);
a condition setting unit (16) which has an analyzing condition for analyzing the information searching result, and supplies the analyzing condition to said analyzing unit (14),
**characterized in that** said analyzing unit (14) executes the analyzing of the information searching result in accordance with the analyzing condition from said condition setting unit (16).

9. The information search system according to claim 8, **characterized by** further comprising a document searching unit (51) which receives the output information output by said search control unit (33), searches a document related to the output information from a database, and outputs a document searching result.

10. The information search system according to claim 9, **characterized by** further comprising a second analyzing unit (61) which receives the document searching result, analyzes the document searching result by extracting predetermined information from the document searching result, and outputs a document analyzing result.

11. The information search system according to claim 10, **characterized by** further comprising a format changing unit (71) which receives the document analyzing result, changes a format of the document analyzing result into a predetermined format, and outputs a document analyzing result whose format is changed.

12. The information search system according to claim 11, **characterized by** further comprising a complementing unit (41) which receives the output information output by said search control unit (33), and complements the output information with information concerning the output information to the output information.

13. The information search system according to claim 12, **characterized in that** the information to be searched is biological information.

14. An information search method **characterized by** comprising the steps of:
receiving a query to a database from a user;
creating a user query for searching information from a database in accordance with the query of the user;
creating a database query for actually searching information from a database in accordance with the user query or a re-search query for re-searching information from a database;
executing a search of information in accordance with the database query;
analyzing an information searching result obtained by the search of information, in accordance with an analyzing condition for analyzing the information searching result;
creating the re-search query by using an information analyzing result which is obtained by analyzing the information searching result;
outputting the information analyzing result as output information, in accordance with an output condition for outputting the information analyzing result.

15. The information search method according to claim 14, **characterized by** further comprising the steps of: receiving conditions concerning an analyzing of the information searching result and an output of the output information, from the user; and holding the conditions as the analyzing condition and the output condition.

16. The information search method according to claim 15, **characterized in that** said outputting the output information includes outputting the output information every time when the analyzing of the information searching result is executed.

17. The information search method according to claim 15, **characterized in that** said outputting the output information includes: holding the information analyzing result which is obtained by each analyzing of the information searching result; and outputting the held information analyzing results in one time, in a case where all re-searches are completed.

18. The information search method according to claim 15, **characterized by** further comprising the steps of: searching a document related to the output information from a document; and outputting a document searching result obtained by the searching.

19. The information search method according to claim 18, **characterized by** further comprising the steps of: analyzing the document searching result by extracting predetermined information from the document searching result; outputting a document analyzing result obtained by analyzing the document searching result.

20. The information search method according to claim 19, **characterized by** further comprising the steps of: changing a format of the document analyzing result into a predetermined format; and outputting a document analyzing result whose format is changed.

21. The information search method according to claim 20, **characterized by** further comprising the step of: complementing the output information with information concerning the output information.

22. The information search method according to claim 21, **characterized in that** the information to be searched is biological information.

23. An information search method **characterized by** comprising the steps of:
receiving a query to a database and a designation of information to be output, from a user;
creating a user query for searching information from a database and a designation information representing the information to be output, in accordance with the query and the designation of the user;
setting a search procedure in accordance with the user query and the designation information, thereby a database query list representing the search procedure is created;
creating a database query for controlling a search of information from the database, in accordance with the database query list and the user query;
executing the search of information in accordance with the database query;
analyzing an information searching result obtained by executing the search, in accordance with an analyzing condition for analyzing the information searching result; and
outputting an information analyzing result obtained by analyzing the information searching result, as output information, in accordance with an output condition for outputting the output information.

24. The information search method according to claim 23, **characterized by** further comprising the steps of: searching a document related to the output information from a database; and outputting a document searching result obtained by the searching.

25. The information search method according to claim 24, **characterized by** further comprising the steps of: analyzing the document searching result by extracting predetermined information from the document searching result; outputting a document analyzing result obtained by analyzing the document searching result.

26. The information search method according to claim 25, **characterized by** further comprising the steps of: changing a format of the document analyzing result into a predetermined format; and outputting a document analyzing result whose format is changed.

27. The information search method according to claim 26, **characterized by** further comprising the step of complementing the output information with information concerning the output information.

28. The information search method according to claim 27, **characterized in that** the information to be searched is biological information.
